# FASCICULE DE BREVET EUROPEEN

(11) **EP 0 745 586 B1**
(45) Date de publication et mention de la délivrance du brevet: **08.03.2000**
(21) Numéro de dépôt: 96401157.1
(22) Date de dépôt: 30.05.1996
(51) Int. Cl.: C07C 275/24, C07C 233/11, C07C 233/22, C07D 209/14, C07D 307/81, C07D 333/58

(54) **Nouveaux aryl(alkyl) propylamides, leur procédé de préparation et les compositions pharmaceutiques qui les contiennent**
Aryl(alkyl)propylamide, Verfahren zu ihrer Herstellung und pharmazeutische Zusammensetzungen, die sie enthalten
Aryl(alkyl) propylamides, process for their preparation and pharmaceutical compositions containing them

(30) Priorité: 31.05.1995 FR 9506433
(43) Date de publication de la demande: 04.12.1996
(73) Titulaire: ADIR ET COMPAGNIE, 92415 Courbevoie Cédex (FR)
(72) Inventeur: Lesieur, Daniel, 59147 Gondecourt (FR); Leclerc, Véronique, 51000 Lille (FR); Depreux, Patrick, 59280 Armentieres (FR); Delagrange, Philippe, 92130 Issy les Moulineaux (FR); Renard, Pierre, 78000 Versailles (FR)

(56) Documents cités:
- EP-A- 0 591 057
- US-A- 3 714 193
- CHEMICAL ABSTRACTS, vol. 116, no. 9, 1992 Columbus, Ohio, US; abstract no. 83585g, XP002012012 & BIOORG. MED. CHEM. LETT., vol. 1, no. 10, 1991, pages 519-522, J.F. RESCH ET AL:
- CHEMICAL ABSTRACTS, vol. 99, no. 19, 1983 Columbus, Ohio, US; abstract no. 158297f, XP002012013 & ARM. KHIM. ZH., vol. 36, no. 5, 1983, pages 317-323, A.K. DURGARYAN ET AL:
- CHEMICAL ABSTRACTS, vol. 92, no. 15, 1980 Columbus, Ohio, US; abstract no. 128645f, XP002012014 & POL. J. PHARMACOL. PHARM., vol. 31, no. 2, 1979, pages 149-156, S. MISZTAL ET AL:
- CHEMICAL ABSTRACTS, vol. 76, no. 3, 1972 Columbus, Ohio, US; abstract no. 14237p, XP002012015 & KHIM. GETEROTSIKL. SOEDIN., vol. 7, no. 9, 1971, pages 1201-1203, I.I. GRANDBERG ET AL:
- CHEMICAL ABSTRACTS, vol. 75, no. 5, 1971 Columbus, Ohio, US; abstract no. 36405y, XP002012016 & KHIM. GETEROTSIKL. SOEDIN., vol. 7, no. 1, 1971, pages 54-57, I.I. GRANDBERG ET AL:
- CHEMICAL ABSTRACTS, vol. 70, no. 1, 1969 Columbus, Ohio, US; abstract no. 3701k, XP002012017 & MONATSH. CHEM., vol. 99, no. 5, 1968, pages 2095-2099, F. SAUTER ET AL:

## Description

L'invention concerne de nouveaux aryl(alkyl)propylamides, leur procédé de préparation et les compositions pharmaceutiques qui les contiennent.

L'invention décrit de nouveaux aryl(alkyl)propylamides qui s'avèrent être de puissants ligands des récepteurs mélatoninergiques.

On connait dans l'état de la technique des dérivés benzothiophène utiles comme intermédiaires de synthèse de dérivés tricycliques antihypertenseurs (US 3,714,193) et des dérivés indoliques utilisés dans la chimie des homotryptamines (Grandberg I.I. et al Khim. Geterotsikl. Soedin. 1971, 7, pp 1201-1203 et 1971, 7, pp 54-57).

On connait également dans l'art antérieur les dérivés N-[3-(4-hydroxy-napht-1-yl)propyl] acétamide et N-[3-(4-benzyloxy-napht-1-yl)propyl]acétamide comme intermédiaires de synthèse (Resch J.F. et al. Bioorg. Med. Chem. Lett. 1991, 1, pp 519-522).

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg 1985, 63, pp 321-341) et d'Alzheimer (Brain Research, 1990, 528, pp 170-174). De même, ces composés ont montré une activité sur certains cancers (Melatonin - clinical Perspectives, Oxford University Press, 1988, page 164-165), sur l'ovulation (Science 1987, 227, pp 714-720) et sur le diabète (Clinical endocrinology, 1986, 24, pp 359-364).

Des composés permettant d'agir sur le système mélatoninergique sont donc pour le clinicien d'excellents médicaments pour le traitement des pathologies liées au système mélatoninergique et notamment celles mentionnées précédemment.

L'invention concerne les composés de formule (I) : 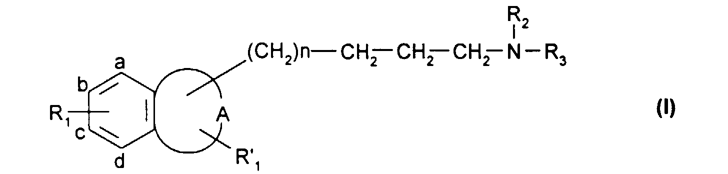 dans laquelle :
- R₁ représente un hydrogène, un hydroxy, un radical R₆ ou un groupement -O-R₆, R₆ étant choisi parmi alkyle, alkyle substitué, cycloalkyle, cycloalkyle substitué, cycloalkylalkyle, cycloalkylalkyle substitué, alcényle, alcynyle, cycloalcényle, phényle, phényle substitué, phénylalkyle, phénylalkyle substitué, dicycloalkylalkyle, dicycloalkylalkyle substitué, diphénylalkyle et diphénylalkyle substitué,
- R'₁ représente un hydrogène, un halogène, ou un groupement choisi parmi :
   - -R'₆,
   - -O-R'₆,
   - -OH,
   - -CO-R₇,
   - -CH₂-R₇,
   - -O-CO-R₇,
      R'₆ étant choisi parmi les mêmes valeurs que R₆, qui est tel que défini précédemment, les radicaux R₆ et R'₆ étant identiques ou différents,
      R₇ représentant un radical choisi parmi (C₁-C₅)alkyle, (C₁-C₅)alkyle substitué, cycloalkyle, cycloalkyle substitué, cycloalkyl(C₁-C₅)alkyle, cydoalkyl(C₁-C₅)alkyle substitué, phényle, phényle substitué, phényl(C₁-C₅)alkyle et phényl(C₁-C₅)alkyle substitué,
- A forme avec le noyau benzénique auquel il est lié un groupement cyclique choisi parmi tétrahydronaphtalène, dihydronaphtalène, naphtalène, benzothiophène, 2,3-dihydrobenzothiophène, benzofurane, 2,3-dihydrobenzofurane, indole et indoline,
- n représente zéro, 1, 2 ou 3,
- R₂ représente un hydrogène ou un alkyle,
- R₃ représente :
   - un groupement de formule (R₃₁): 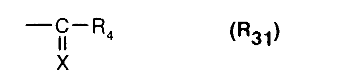 avec X représentant un soufre ou un oxygène et R₄ représentant un hydrogène ou un radical choisi parmi alkyle, alkyle substitué, alcényle, alcynyle, cycloalkyle, cycloalkyle substitué, cycloalkylalkyle et cycloalkylalkyle substitué,
   - ou un groupement de formule (R₃₂): 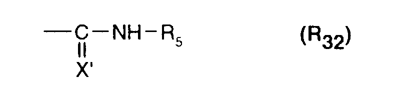 avec X' représentant un soufre ou un oxygène et R₅ représentant un hydrogène ou un radical non substitué ou substitué choisi parmi alkyle, cycloalkyle, et cycloalkylalkyle,
      le composé de formule (I) ne pouvant pas représenter le N-[3-(4-hydroxy-napht-1-yl) propyl]acétamide ou le N-[3-(4-benzyloxy-napht-1-yl)propyl]acétamide,
   étant entendu que :
   - lorsque A forme avec le noyau benzénique auquel il est lié un groupement benzothiophène, n est égal à zéro, R'₁ et R₂ représentent simultanément un atome d'hydrogène, R₁ représente un atome d'hydrogène, un groupement hydroxy un groupement alkyle ou un groupement alkoxy et X représente un atome d'oxygène, alors R₄ ne peut représenter un atome d'hydrogène ou un groupement alkyle,
   - lorsque A forme avec le noyau benzénique auquel il est lié un groupement benzothiophène, R₄ est un radical alkyle ou vinyle, R₁ est un hydrogène ou un méthyle et R'₁ est un hydrogène, alors n est différent de zéro,
   - lorsque A forme avec le noyau benzènique auquel il est lié un groupement indole, n est égal à zéro, R₂ représente un hydrogène, R'₁ représente un hydrogène ou radical phényle, méthyle ou benzyle, substitué sur l'azote du groupement indole formé par A, et R₁ représente un hydrogène ou un groupement méthyle alors R₄ ne peut pas être un radical méthyle ou éthyle,
   - le terme "alkyle" désigne un groupement linéaire ou ramifié contenant de 1 à 6 atomes de carbone,
   - les termes "alcényle" et "alcynyle" désignent des groupements linéaires ou ramifiés contenant de 2 à 6 atomes,
   - le terme "cycloalkyle" désigne un groupement de 3 à 8 atomes de carbone,
   - le terme "cycloalcényle" désigne un groupement insaturé de 5 à 8 atomes de carbone,
   - le terme "substitué" associé au radical "alkyle" signifie que ce radical est substitué par un ou plusieurs substituants choisis parmi halogène, hydroxy et alkoxy,
   - le terme "substitué" associé aux radicaux cycloalkyle, cycloalkylalkyle, dicycloalkylalkyle signifie que ces radicaux sont substitués sur la partie cycloalkyle par un ou plusieurs groupements choisis parmi halogène, alkyle, hydroxy, alkoxy et oxo,
   - et le terme "substitué" associé aux radicaux phényle, phénylalkyle, phényl(C₁-C₅)alkyle, diphénylalkyle signifie que ces radicaux sont substitués sur la partie phényle par un ou plusieurs groupements choisis parmi halogène, hydroxy, alkyle, alkyle substitué par un ou plusieurs halogènes, et alkoxy,
   leurs énantiomères et diastéréoisomères,
   et leurs sels d'addition avec une base pharmaceutiquement acceptable.

L'invention concerne particulièrement les composés de formule (I) dans laquelle, pris séparément ou ensemble,
- R₁ représente un alkyle,
- R₁ représente un alkoxy,
- A forme avec le noyau benzènique auquel il est lié un tétrahydronaphtalène,
- A forme avec le noyau benzènique auquel il est lié un naphtalène,
- A forme avec le noyau benzènique auquel il est lié un dihydronaphtalène,
- A forme avec le noyau benzénique auquel il est lié un benzofurane,
- A forme avec le noyau benzènique auquel il est lié un benzothiophène,
- A forme avec le noyau benzènique auquel il est lié un indole,
- R₂ représente un hydrogène,
- R₂ représente un alkyle,
- R₃ représente un groupement R₃₁ tel que défini dans la formule (I),
- R₃ représente un groupement R₃₂ tel que défini dans la formule (I),
- R₄ représente un atome d'hydrogène,
- R₄ représente un alkyle,
- R₄ représente un cycloalkyle,
- R₄ représente un alcényle,
- R₅ représente un hydrogène,
- R₅ représente un alkyle,
- R₅ représente un cycloalkyle,
- X représente un oxygène,
- X représente un soufre,
- X' représente un oxygène,
- X' représente un soufre,
- R'₁ représente un hydrogène,
- R'₁ représente un alkyle,
- R'₁ représente un phénylalkyle,
- n représente zéro,
et n représente 1.

Par exemple, l'invention concerne les composés particuliers de formule (I) répondant aux formules respectives (1) à (12) : 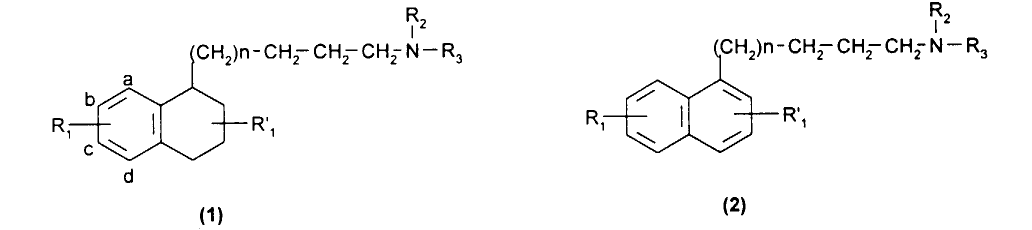 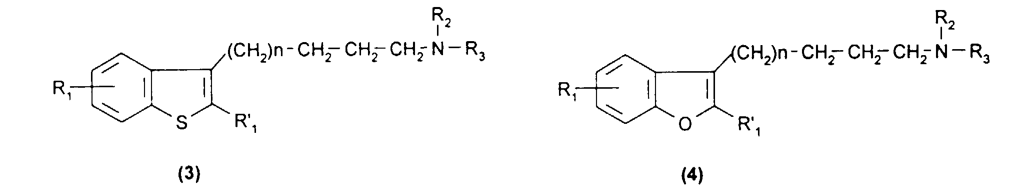 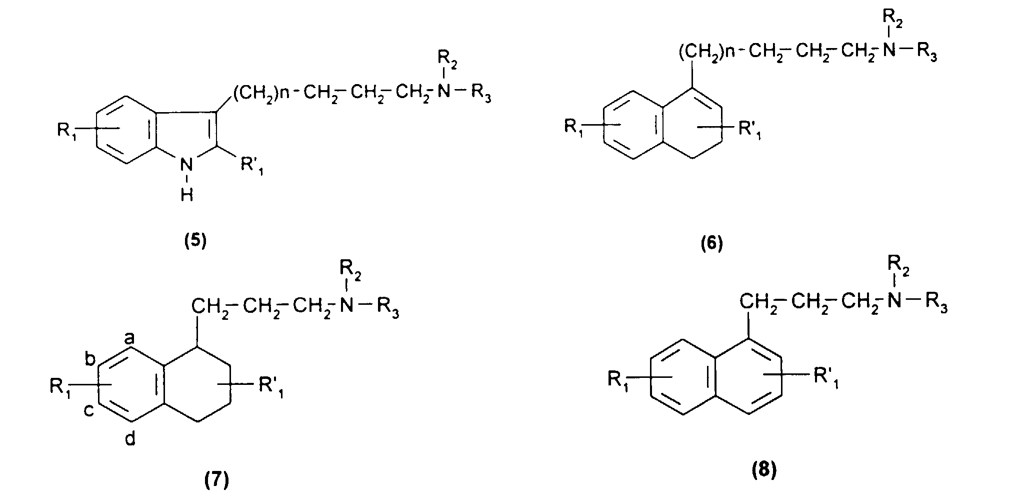 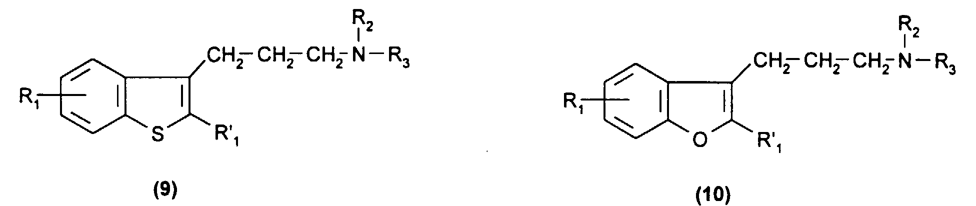 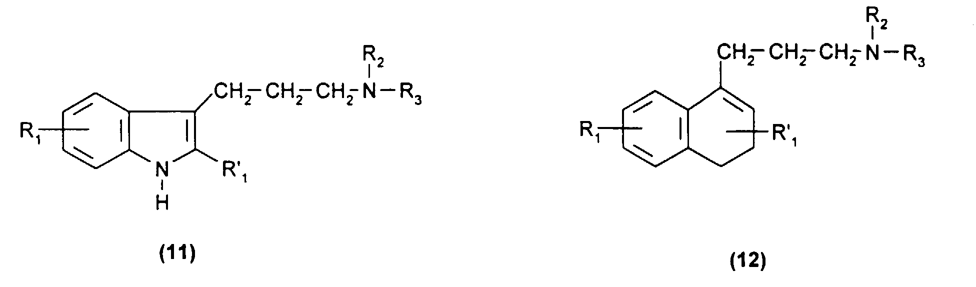 dans lesquelles R₁, R'₁, R₂, R₃ et n sont tels que défini dans la formule (I).

L'invention concerne particulièrement les composés de formule (I) dans laquelle R₁ est:
- en position a du noyau benzènique,
- en position b du noyau benzénique,
- en position c du noyau benzènique,
- ou en position d du noyau benzènique.

Par exemple, l'invention concerne les composés de formule (I) dans laquelle R₁ est en position b du noyau benzénique.

L'invention concerne particulièrement les composé de formule (I) dans laquelle R'₁ est un hydrogène.

Par exemple, l'invention concerne les composés suivants :
- N-[3-(7-méthoxynapht-1-yl)propyl]acétamide,
- N-[3-(7-méthoxynapht-1-yl)propyl]pentanamide,
- N-[3-(7-méthoxynapht-1-yl)propyl]cyclopropanecarboxamide,
- N-propyl N'-[3-(7-méthoxynapht-1-yl)propyl]urée,

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle, ou hexyle, et les isomères de squelette des radicaux pentyle ou hexyle ;
les radicaux alcényles présents dans la formule (I) peuvent être choisis parmi vinyle, allyle, propényle, butényle, pentényle et hexényle, ainsi que leurs isomères suivant la position de la double liaison ;
les radicaux alcynyles présents dans la formule (I) peuvent être choisis parmi éthynyle, propargyle, butynyle, pentynyle et hexynyle, ainsi que leurs isomères suivant la position de la triple liaison ;
les halogènes présents dans la formule (I) peuvent être choisis parmi le brome, le chlore, le fluor, et l'iode ;
les cycloalkyles présents dans la formule (I) peuvent être choisis parmi cyclopropyle, cyclobutyle, cyclopentyle, cyclohexyle, cycloheptyle et cyclooctyle;
les cycloalcényles présents dans la formule (I) peuvent être choisis parmi cyclopropényle, cyclobutényle, cyclopentényle, cyclohexényle, cycloheptényle et cyclooctényle.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

L'invention concerne également le procédé de préparation des composés de formule (I) caractérisé en ce que on fait réagir un composé de formule (II) 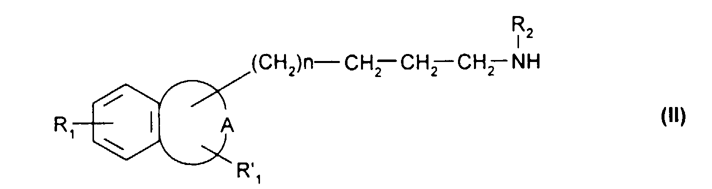 dans laquelle R₁, R'₁, R₂, n et A sont tels que définis dans la formule (I),
- soit avec un composé de formule (IIIa) ou (IIIb) : 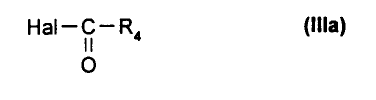 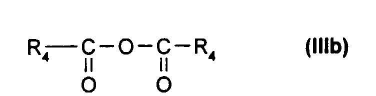 l'anhydride de formule (IIIb) étant mixte ou symétrique,
   dans laquelle R₄ est tel que défini dans la formule (I) et Hal représente un halogène, afin d'obtenir les composés de formule (I/a) : 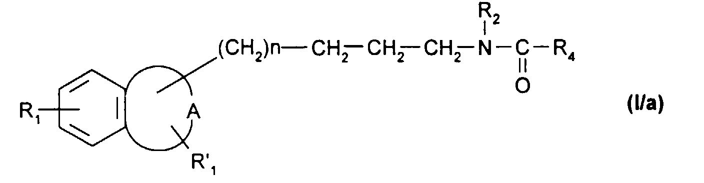 dans laquelle R₁, R'₁, R₂, R₄, A et n sont tels que définis précédemment,
   composés de formule (I/a) qui sont soumis à un agent de thionation, par exemple le réactif de Lawesson, pour obtenir les composés de formule (I/a): 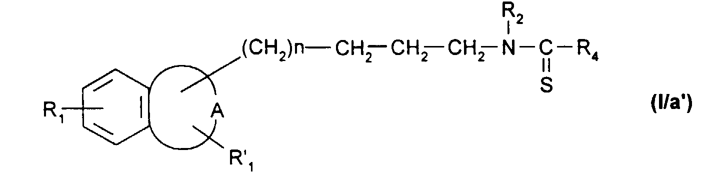 dans laquelle R₁, R'₁, R₂, R₄, A et n sont tels que définis précédemment,
- soit avec un composé de formule (IV) :

   X'=C=N-R₅ **(IV)**

   dans laquelle X' et R₅ sont tels que définis dans la formule (I)
   afin d'obtenir les composés de formule (I/b) : 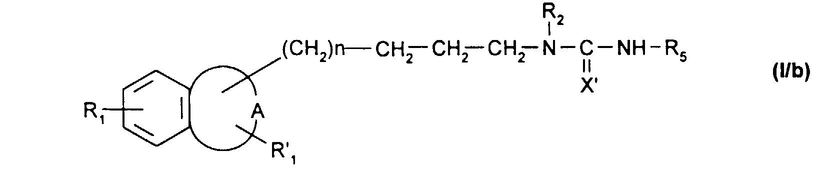 dans laquelle R₁, R'₁, R₂, R₅, A, n, et X' sont tels que définis précédemment,
   les composés de formule (I/a), ((I/a') et (I/b) formant l'ensemble des composés de formule (I), pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.
   Par exemple, l'invention s'étend au procédé de préparation des composés de formule (I/c): 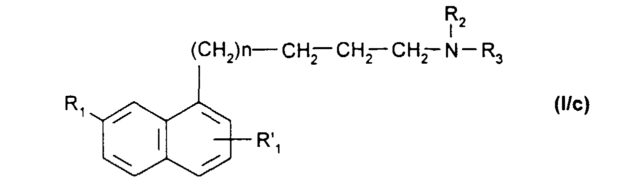 dans laquelle R₁, R'₁, R₂, R₃ et n sont tels que définis dans la formule (I), caractérisé en ce que :
   on fait réagir un composé de formule (II/a): 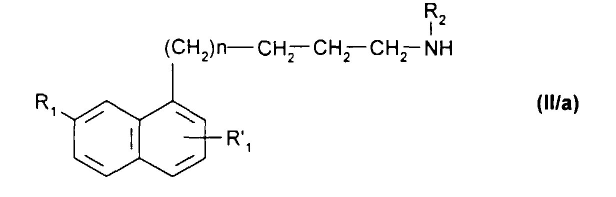 dans laquelle R₁, R'₁, R₂ et n sont tels que définis précédemment,
- soit avec un composé de formule (IIIa) ou (IIIb) tels que définis précédemment, afin d'obtenir les composés de formule (I/d) : 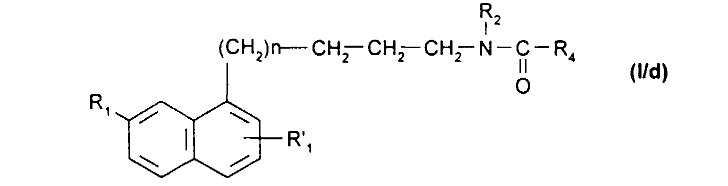 dans laquelle n, R₁, R'₁, R₂, et R₄ sont tels que définis précédemment, qui sont ensuite soumis à un agent de thionation, par exemple le réactif de Lawesson, pour obtenir les composés de formule (I/d') : 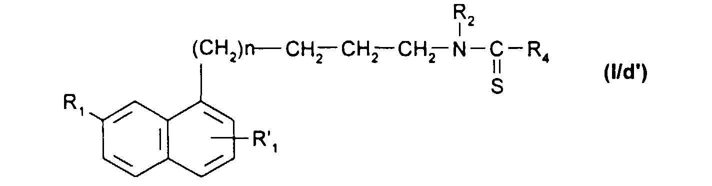 dans laquelle n, R₁, R'₁, R₂, et R₄ sont tels que définis précédemment,
- soit avec un composé de formule (IV) tel que défini précédemment,
   afin d'obtenir les composés de formule (I/e) : 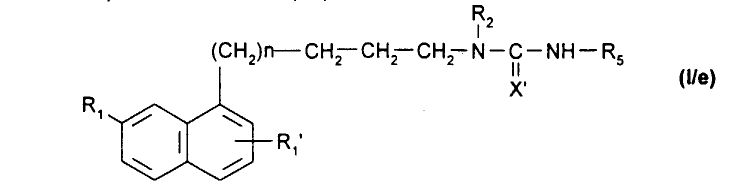 dans laquelle n, R₁, R'₁, R₂, R₅ et X' sont tels que définis précédemment,
   les composés de formule (I/d), (I/d') et (I/e) formant l'ensemble des composés de formule (I/c), composés de formule (I/c) pouvant être si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

L'invention s'étend également au procédé de préparation des composés de formule (I/g), (I/h), (I/i) et (I/j), cas particuliers des composés de formule (I), caractérisé en ce qu'on fait réagir un composé de formule (I/f): 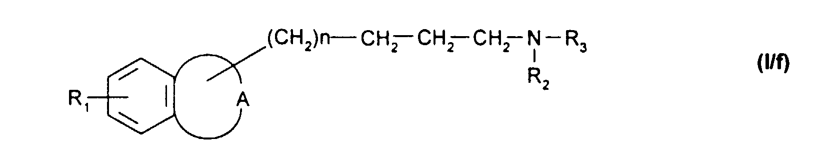 dans laquelle n, A, R₁, R₂ et R₃ sont tels que définis dans la formule (I),
avec un composé de formule (V): 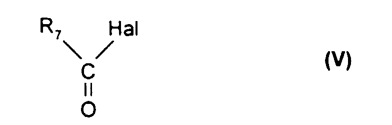 dans laquelle Hal est un atome d'halogène et R₇ est tel que défini dans la formule (I), pour obtenir un composé de formule (I/g): 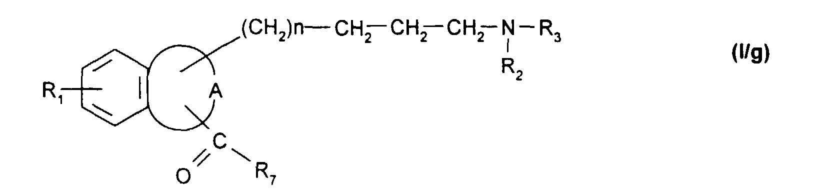 dans laquelle n, A, R₁, R₂, R₃ et R₇ sont tels que définis précédemment,
qui peut être, si on le désire,
- soit soumis à une oxydation par une réaction de Baeyer-Villiger pour obtenir un composé de formule (I/h) correspondant : 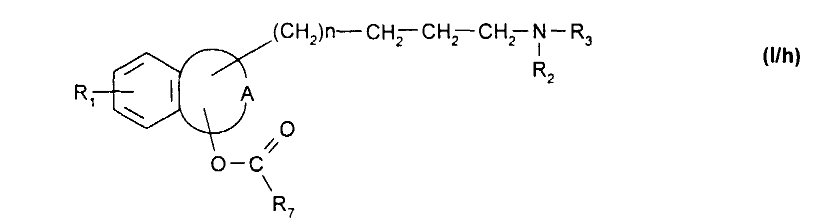 dans laquelle n, A, R₁, R₂, R₃ et R₇ sont tels que définis précédemment,
   qui peut être soumis à une saponification en présence d'hydroxyde de sodium, pour obtenir un composé de formule (I/i) correspondant : 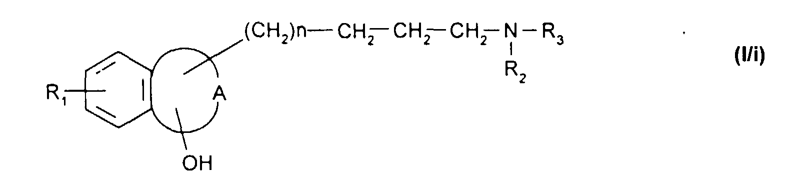 dans laquelle n, A, R₁, R₂, R₃ sont tels que définis précédemment,
- soit soumis à une réduction pour obtenir un composé de formule (I/j): 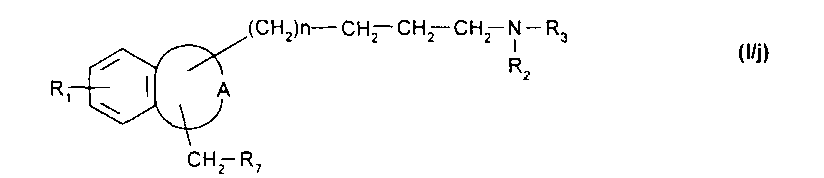 dans laquelle n, A, R₁, R₂, R₃ et R₇ sont tels que définis précédemment,
   les composés de formule (I/f), (I/g), (I/h), (I/i) et (I/j) pouvant être si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

Les matières premières utilisées dans les procédés précédemment décrits sont soit commerciales, soit aisément accessibles à l'homme du métier d'après la littérature et les exemples de préparations données ci-après.

Ainsi les composés de formule (Il) sont aisément accessibles à l'homme du métier par réaction d'un acide carboxylique de formule (II/a) : 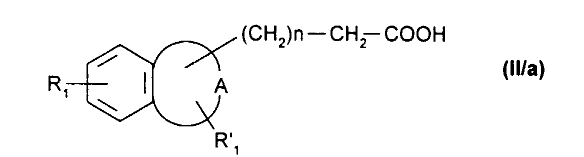 dans laquelle n, A, R₁ et R'₁ sont tels que définis dans la formule (I),
1) soit avec du méthanol en présence de chlorure de thionyle pour conduire à un dérivé de formule (II/b) : 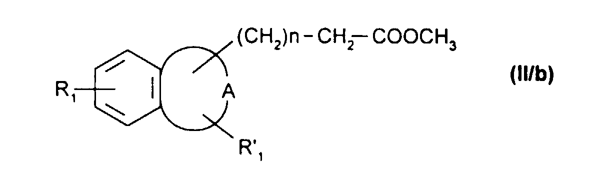 dans laquelle n, A, R₁ et R'₁ sont tels que définis précédemment,
   qui est réduit en un dérivé de formule (Il/c) : 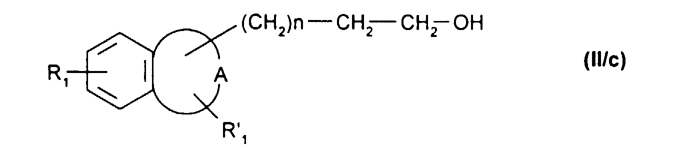 dans laquelle n, A, R₁ et R'₁ sont tels que définis précédemment,
   qui, mis en réaction avec du chlorure de méthanesulfonyle, conduit à un dérivé de formule (II/d) : 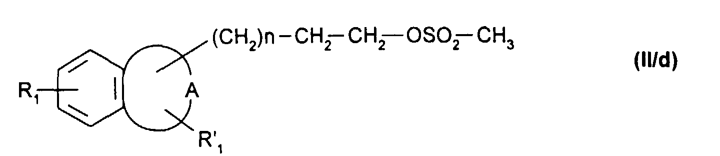 dans laquelle n, A, R'₁ et R₁ sont tels que définis précédemment,
2) soit avec de la diméthylamine, pour conduire à un dérivé de formule (Il/e) : 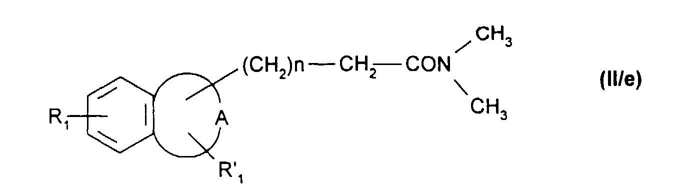 dans laquelle n, A, R₁ et R'₁ sont tels que définis précédemment,
   qui est alors réduit en un dérivé de formule (II/f) : 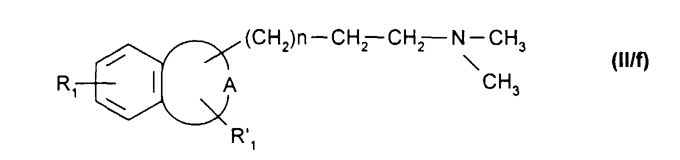 dans laquelle n, A, R₁ et R'₁ sont tels que définis précédemment,
   qui est soumis à l'action de l'iodure de méthyle pour conduire à un dérivé de formule (II/g) : 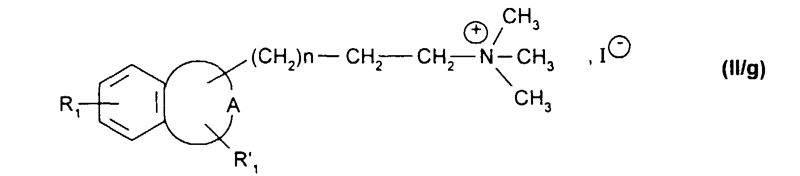 dans laquelle n, A, R₁ et R'₁ sont tels que définis précédemment,
   composés de formule (II/d) et (II/g), qui sont, l'un ou l'autre, mis en réaction avec du cyanure de potassium pour donner des dérivés de formule (II/h): 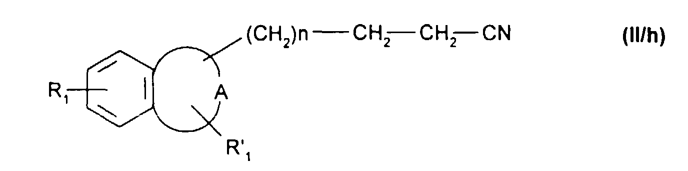 dans laquelle n, A, R₁ et R'₁ sont tels que définis précédemment,
   qui est ensuite hydrogéné pour conduire à un composé de formule (Il/i) : 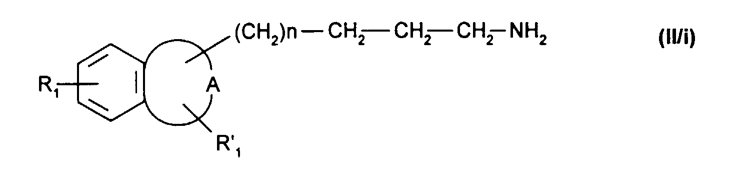 dans laquelle n, A, R₁ et R'₁ sont tels que définis précédemment,
   qui est enfin éventuellement alkylé en un composé de formule (II/j) : 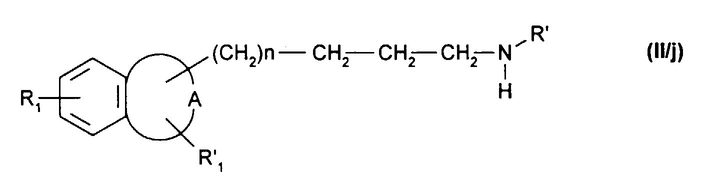 dans laquelle n, A, R₁ et R'₁ sont tels que définis précédemment et R' est un groupement alkyle,
   les composés (Il/i) et (Il/j) formant l'ensemble des composés de formule (Il) : 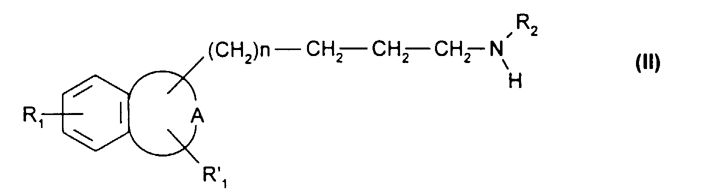 dans laquelle n, A, R₁, R'₁ et R₂ sont tels que définis dans la formule (I),
   qui peuvent être séparés en leurs énantiomères ou diastéréoisomères et salifiés par un acide pharmaceutiquement acceptable.

Par exemple, les composés de formule (Il) dans lesquels n est égal à zéro sont aisément accessibles à l'homme de métier, par le procédé de préparation exposé ci-dessus.

Il est aussi possible de préparer les composés de formule (Il/k) : 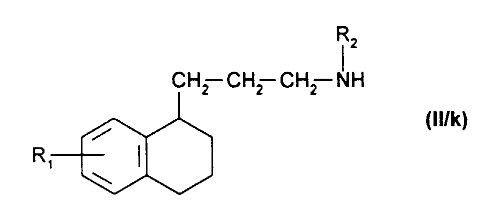 dans laquelle R₁ et R₂ sont tels que définis dans la formule (I),
par réaction d'un composé de formule (VI) : 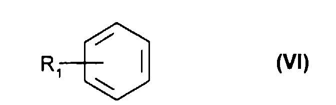 dans laquelle R₁ est tel que défini précédemment,
avec l'anhydride succinique pour obtenir un composé de formule (VII) : 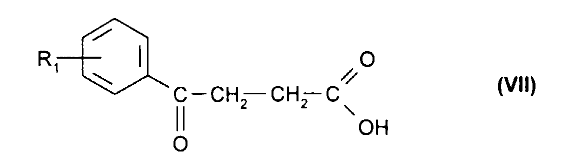 dans laquelle R₁ est tel que défini précédemment,
qui est réduit pour obtenir un composé de formule (VIII) : 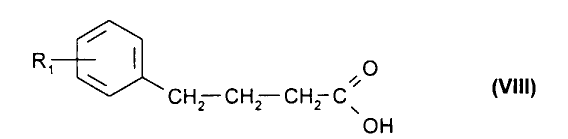 dans laquelle R₁ est tel que défini précédemment,
qui est ensuite cyclisé pour obtenir un composé de formule (IX) : 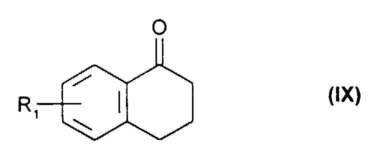 dans laquelle R₁ est tel que défini précédemment,
qui est mis en réaction avec du cyanoéthyl phosphonate diéthylique pour obtenir le composé de formule (X) : 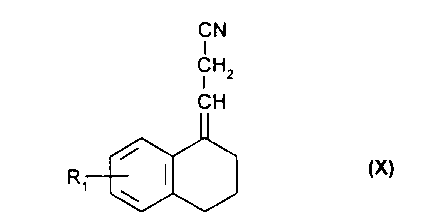 dans laquelle R₁ est tel que défini précédemment,
qui est ensuite hydrogéné pour obtenir le composé de formule (II/ℓ) : 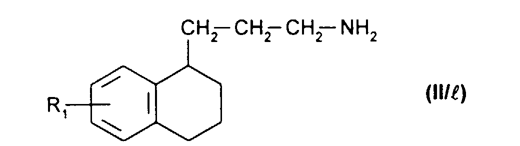 dans laquelle R₁ est tel que défini précédemment,
composé de formule (II/ℓ) éventuellement alkylé sur la fonction amine pour obtenir un composé de formule (II/m): 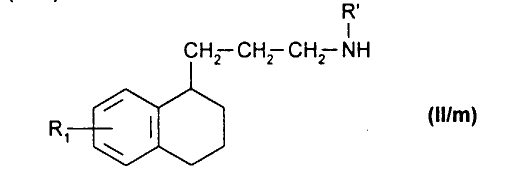 dans laquelle R₁ est tel que défini précédemment et R' représente un groupement alkyle, les composés de formule (II/ℓ) et (II/m) formant l'ensemble des composés de formule (Il/j).

L'aromatisation des composés à structure tétrahydronaphtalénique tels que décrits ci-dessus permet d'obtenir les composés utiles pour la préparation des composés de formule (I) dans lesquels A forme avec le noyau benzènique auquel il est lié un groupement naphtalène.

Inversement, la réduction des composés de formule (I/c) à structure naphtalénique permet d'obtenir les composés de formule (I) dans lesquels A forme avec le noyau benzènique auquel il est lié un groupement dihydronaphtalène ou tetrahydronaphtalène, ou les composés utiles pour leur préparation.

Les composés intermédiaires de formule (II/f): 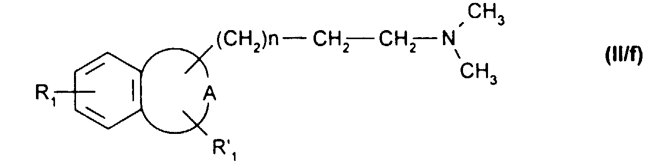 dans laquelle A, n, R₁ et R'₁ sont tels que définis dans la formule (I),
peuvent aussi être préparés par action du formaldéhyde sur des amines de formule (Il/n) : 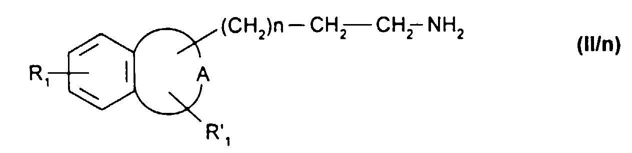 dans laquelle A, n, R₁ et R'₁ sont tels que définis précédemment.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique et des troubles liés au système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, du psoriasis, de l'insomnie, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que des troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant les produits de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement entre 1 à 100 mg, par exemple entre 1 à 10 mg.

Les exemples suivants illustrent l'invention, mais ne la limitent en aucune façon.

### PRFPARATION 1: 3-(7-METHOXY NAPHT-1-YL)PROPYLAMINE CHLORHYDRATE

### VOIE 1 :

### STADE A: (7-METHOXY NAPHT-1-YL)-N,N-DIMETHYLACETAMIDE

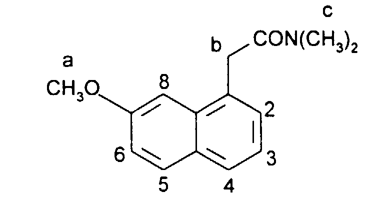

| **Réactifs :** | |
|---|---|
| Acide (7-méthoxy napht-1-yl)acétique | 0,032 mole (7 g) |
| Chlorure de thionyle | 0,128 mole (9,5 cm³) |
| Diméthylamine (solution aqueuse à 25 %) | 0,390 mole (70 cm³) |
| Chlorofome | 300 cm³ |
| Ether anhydre | 200 cm³ |
| Solution aqueuse de soude à 5 % | 20 cm³ |

### Mode opératoire :

Dans un ballon de 750 cm³, dissoudre 7 g d'acide (7-méthoxy napht-1-yl)acétique dans 150 cm³ de chloroforme. Porter à reflux puis ajouter lentement 9,5 cm³ de chlorure de thionyle dilué dans 150 cm³ de chloroforme. Laisser à reflux pendant 2 heures. Laisser refroidir, évaporer sous vide le solvant. Dissoudre le résidu huileux dans de l'éther anhydre. Placer le ballon dans un bain de glace à -5°C, et ajouter lentement 70 cm³ de diméthylamine en solution aqueuse à 25 %. Laisser sous agitation pendant 1 heure et filtrer le précipité qui s'est formé puis le recristalliser dans le solvant approprié.

| **Caractéristiques :** | |
|---|---|
| Masse molaire | 243,31 g/mol pour C₁₅H₁₇NO₂ |
| Point de fusion | 86-88°C |
| Solvant de recristallisation | hexane |
| Rendement | 31 % |
| Solvant d'élution | Acétate d'éthyle |
| Rf | 0,32 |
| Aspect | cristaux blancs |

| **Analyse élémentaire :** | | | |
|---|---|---|---|
| | **C%** | **H%** | **N%** |
| Calculé | 74,04 | 7,04 | 5,76 |
| Trouvé | 74,14 | 7,24 | 5,84 |

### Analyse spectroscopique dans l'infra-rouge:

- 3000-2810 cm⁻¹ : ν CH (alkyles)
- 1650-1570 cm⁻¹ : ν CO (amide)
- 1260 cm⁻¹ : ν OCH₃

| **Analyse spectroscopique de RMN du proton (CDCl3, δ, 80 MHz) :** | |
|---|---|
| 3,00 ppm (singulet, 6H) | H_{c} |
| 3,95 ppm (singulet, 3 H) | Hₐ |
| 4,10 ppm (singulet, 2H) | H_{b} |
| 7,10-7,40 ppm (massif, 4H) | H_{2,3,6,8} |
| 7,60-7,90 ppm (massif, 2H) | H_{4,5} |

### STADE B : CHLORHYDRATE DE LA N-[2-(7-METHOXY NAPHT-1-YL)ETHYL]-N,N-DIMETHYLAMINE

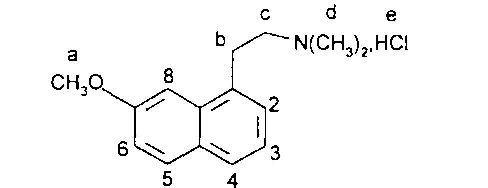

### 1) A partir de la (7-méthoxy napht-1-yl) N.N-diméthylacétamide

| **Réactifs :** | |
|---|---|
| (7-méthoxy napht-1-yl) N,N-diméthylacétamide | : 0,004 mole (1 g) |
| Hydrure de lithium et d'aluminium : | 0,053 mole (2 g) |
| Ether anhydre : | 150 cm³ |

### Mode opératoire :

Dissoudre, dans une fiole de 100 cm³, 1 g de (7-méthoxy napht-1-yl) N,N-diméthylacétamide dans l'éther. Ajouter l'hydrure de lithium et d'aluminium. Porter à reflux pendant 3 heures. Ajouter goutte à goutte la quantité nécéssaire de solution aqueuse de soude à 10 % pour hydrolyser l'excès d'hydrure n'ayant pas réagi. Filtrer le milieu réactionnel. Extraire la phase éthérée par 2 fois 20 cm³ d'une solution de soude, la laver à l'eau, la sécher sur sulfate de sodium et l'évaporer sous pression réduite. Dissoudre le résidu dans l'éther anhydre et y ajouter de l'éther anhydre saturé en HCI, filtrer le précipité et le recristalliser.
Rendement : 83 %

### 2) A partir du chlorhydrate de la 2-(7-méthoxy napht-1-yl)éthylamine

| **Réactifs :** | |
|---|---|
| Chlorhydrate de 2-(7-méthoxy napht-1-yl)éthylamine | 0,042 mole (10 g) |
| Formaldéhyde à 30 % | 0,084 mole (8,5 cm³) |
| Nickel de Raney | 2 g |
| Méthanol anhydre | 200 cm³ |
| Carbonate de sodium | 0,042 mole (4,46 g) |

### Mode opératoire :

Dans une fiole de 250 cm³, dissoudre 10 g de chlorhydrate de la 2-(7-méthoxy napht-1-yl) éthylamine dans 100 cm³ d'eau, puis ajouter petit à petit 4,46 g de carbonate de sodium. Extraire la phase aqueuse par 4 fois 50 cm³ de chloroforme, laver les phases organiques, les sécher sur sulfate de magnésium et évaporer le solvant. Reprendre le résidu dans 200 cm³ de méthanol anhydre, ajouter 2 g de nickel de Raney, 8,5 cm³ de formaldéhyde à 30 %. Mettre l'autoclave sous pression d'hydrogène de 50 bars pendant 24 heures. Filtrer le milieu réactionnel. Evaporer le solvant. Reprendre le résidu par de l'acétate d'éthyle, laver cette phase organique par de l'eau, la sécher sur sulfate de magnésium puis l'évaporer. Dissoudre le résidu huileux dans l'éther et ajouter de l'éther anhydre saturé en HCI. Essorer le précipité obtenu et recristalliser.
Rendement : 24 %

| **Caractéristiques :** | |
|---|---|
| Masse molaire | 265,78 g/mol pour C₁₅H₂₀NOCl |
| Point de fusion | 142-144°C |
| Solvant de recristallisation | acétone |
| Solvant d'élution | Acétone-Toluène-Cyclohexane (4-4-2) + 1 cm³ de triéthylamine |
| Rf | 0,41 |

**Analyse spectroscopique dans l'infra-rouge:**
- 3020-2820 cm⁻¹ : ν CH (alkyles)
- 2700-2300 cm⁻¹ : ν NH⁺ (sel d'amine tertiaire)
- 1610 et 1590 cm⁻¹ : ν C=C (aromatiques)
- 1250 cm⁻¹ : ν OCH₃

| **Analyse spectroscopique de RMN du proton (CDCl**_{**3**}**, δ, 80 MHz) :** | |
|---|---|
| 2,00 ppm (singulet, 1H) | Hₑ, échangeable dans D₂O |
| 2,90 ppm (doublet, 6H) | H_{d} J = 4,70 Hz |
| 3,30 ppm (multiplet, 2H) | H_{b} |
| 3,65 ppm (multiplet, 2H) | H_{c} |
| 4,05 ppm (singulet, 3H) | Hₐ |
| 7,05-7,40 ppm (massif, 4H) | H_{2,3,6,8} |
| 7,40-7,80 ppm (massif, 2 H) | H_{4,5} |

### STADE C : IODURE DE N,N,N,-TRIMETHYL-N-2-(7-METHOXY NAPHT-1-YL) ETHYLAMMONIUM

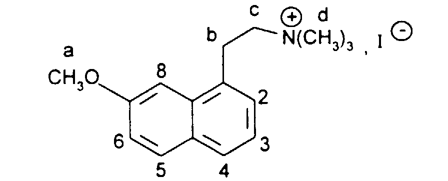

| **Réactifs :** | |
|---|---|
| Chlorhydrate de la N-2-(7-méthoxy napht-1-yl)éthyl-N,N-diméthylamine | 0,008 mole (2,13 g) |
| Iodure de méthyle | 0,080 mole (11,4 g) |
| Carbonate de sodium | 0,008 mole (0,8 g) |
| Ethanol | 100 cm³ |

### Mode opératoire :

Dissoudre 2,13 g du chlorhydrate de la N-2-(7-méthoxy napht-1-yl)éthyl-N,N-diméthylamine dans de l'eau, ajouter petit à petit 0,8 g de carbonate de sodium. Extraire la phase aqueuse par 3 fois 50 cm³ de chloroforme, laver la phase organique par l'eau, la sécher sur sulfate de magnésium et évaporer. Reprendre le résidu dans l'éthanol (50 cm³) et ajouter l'amine à une solution éthanolique glacée contenant 11,4 g d'iodure de méthyle. Laisser dans un bain de glace pendant 24 heures. Filtrer le précipité obtenu et le recristalliser.

| **Caractéristiques :** | |
|---|---|
| Masse molaire | 371,26 g/mol pour C₁₆H₂₂NOI |
| Point de fusion | 232-234°C |
| Solvant de recristallisation | alcool 95° |
| Rendement | 92 % |

### Analyse spectroscopique dans l'infra-rouge:

- 3020-2800 cm⁻¹ : ν CH (alkyles)
- 1610 et 1590 cm⁻¹ : ν C=C (aromatiques)
- 1250 cm⁻¹ : ν OCH₃

| **Analyse spectroscopique de RMN du proton (DMSO d**_{**6**}**, δ, 80 MHz) :** | |
|---|---|
| 3,30 ppm (singulet, 9H) | H_{d} |
| 3,60 ppm (massif, 4H) | H_{b,c} |
| 3,95 ppm (singulet, 3H) | Hₐ |
| 7,15-7,60 ppm (massif, 4H) | H_{2,3,6,8} |
| 7,70-8,00 ppm (massif, 2H) | H_{4,5} |

### VOIE 2 :

### STADE A': (7-METHOXY NAPTH-1-YL)ACETATE DE METHYLE

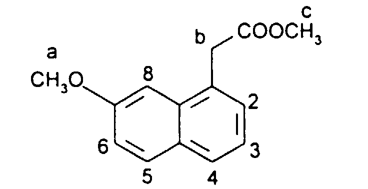

| **Réactifs :** | |
|---|---|
| Acide (7-méthoxy napht-1-yl)acétique | 0,023 mole (5 g) |
| Chlorure de thionyle | 0,092 mole (6,75 cm³) |
| Méthanol | 150 cm³ |

### Mode opératoire :

Dans une fiole de 250 cm³, dissoudre 5 g d'acide (7-méthoxy napht-1-yl)acétique dans le méthanol. Refroidir dans un bain de glace à -10°C. Ajouter, sous agitation, goutte à goutte, à l'aide d'une ampoule à brome, 6,75 cm³ de chlorure de thionyle. Laisser sous agitation pendant 30 minutes. Evaporer le milieu réactionnel sous pression réduite. Dissoudre le résidu huileux dans de l'acétate d'éthyle, extraire la phase organique par 3 fois 50 cm³ d'une solution de carbonate de potassium à 10%, puis à l'eau, la sécher sur sulfate de sodium et l'évaporer. Recristalliser le résidu huileux obtenu qui se solidifie à température ambiante.

| **Caractéristiques :** | |
|---|---|
| Masse molaire | 230,26 g/mol pour C₁₄H₁₄O₃ |
| Point de fusion | 51-52°C |
| Solvant de recristallisation | n-hexane |
| Rendement | 94% |
| Solvant d'élution | Acétone-Toluène-Cyclohexane (4-4-2) |
| Rf | 0,73 |

| **Analyse élémentaire :** | | | |
|---|---|---|---|
| | **C%** | **H%** | **N%** |
| Calculé | 73,03 | 6,13 | 20,85 |
| Trouvé | 72,86 | 6,18 | 20,75 |

### Analyse spectroscopique dans l'infra-rouge:

- 3040-2820 cm⁻¹ : ν CH (alkyles)
- 1730 cm⁻¹ : ν CO (ester)
- 1620 et 1600 cm⁻¹ : ν C=C (aromatiques)
- 1260 cm⁻¹ : ν OCH₃

| **Analyse spectroscopique de RMN du proton (CDCl**_{**3**}**, δ, 80 MHz) :** | |
|---|---|
| 3,65 ppm (singulet, 3H) | H_{c} |
| 3,90 ppm (singulet, 3H) | Hₐ |
| 4,00 ppm (singulet, 2H) | H_{b} |
| 7,10-7,45 ppm (massif 4H) | H_{2,3,6,8} |
| 7,60-7,90 ppm (massif, 2H) | H_{4,5} |

### STADE B': 2-(7-METHOXY NAPTH-1-YL)ETHANOL

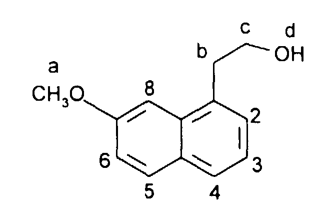

| **Réactifs :** | |
|---|---|
| (7-méthoxy napht-1-yl)acétate de méthyle | 0,022 mole (5 g) |
| Hydrure de lithium et d'aluminium | 0,088 mole (3,34 g) |
| Ether anhydre | 100 cm³ |

### Mode opératoire :

Dans une fiole de 250 cm³, mettre 3,34 g d'hydrure de lithium et d'aluminium, ajouter doucement l'éther sous agitation. A l'aide d'une ampoule à brome, ajouter lentement 5 g de (7-méthoxy napht-1-yl)acétate de méthyle préalablement dissous dans 50 cm³ d'éther. Laisser agiter pendant 30 minutes. Verser le milieu réactionnel très lentement dans un mélange d'eau et de glace afin d'hydrolyser l'excès d'hydrure n'ayant pas réagi. Filtrer le milieu réactionnel. Extraire la phase aqueuse par 3 fois 50 cm³ d'éther, sécher les phases éthérées sur sulfate de magnésium et les évaporer. Recristalliser le solide blanc obtenu.

| **Caractéristiques :** | |
|---|---|
| Masse molaire | 202,25 g/mol pour C₁₃H₁₄O₂ |
| Point de fusion | 80-82°C |
| Solvant de recristallisation | cyclohexane |
| Rendement | 82% |
| Solvant d'élution | Acétone-Toluène-Cyclohexane (4-4-2) |
| Rf | 0,34 |
| Aspect | Poudre blanche |

| **Analyse élémentaire :** | | | |
|---|---|---|---|
| | **C%** | **H%** | **O%** |
| Calculé | 77,20 | 6,98 | 15,82 |
| Trouvé | 76,91 | 7,13 | 15,71 |

| **Analyse spectroscopique dans l'infra-rouge:** | |
|---|---|
| 3300-3100 cm⁻¹ | ν OH, large bande |
| 3000-2800 cm⁻¹ | ν CH (alkyles) |
| 1610 et 1590 cm⁻¹ | ν C=C (aromatiques) |
| 1250 cm⁻¹ | ν OCH₃ |
| 1030 cm⁻¹ | ν C-O (alcool) |

| **Analyse spectroscopique de RMN du proton (CDCl**_{**3**}**, δ, 80 MHz) :** | |
|---|---|
| 1,60 ppm (singulet, 1H) | H_{d}, échangeable dans D₂O |
| 3,30 ppm (triplet, 2H) | H_{b} J_{b-c} = 7,10 Hz |
| 3,90 ppm (singulet, 3H) | Hₐ |
| 4,00 ppm (triplet, 2H) | H_{c} J_{c-b} = 7,10 Hz |
| 7,00-7,40 ppm (massif, 4H) | H_{2,3,6,8} |
| 7,60-7,80 ppm (massif, 2H) | H_{4,5} |

### STADE C' : MESYLATE DU 2-(7-METHOXY NAPHT-1-YL)ETHANOL

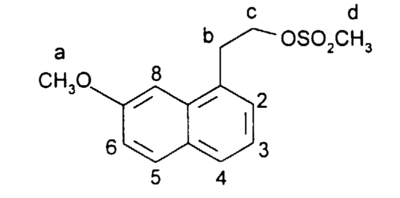

| **Réactifs :** | |
|---|---|
| 2-(7-méthoxy napht-1-yl)éthanol | 0,020 mole (4 g) |
| Chlorure de mésyle | 0,024 mole (2,75 g) |
| Triéthylamine | 0,024 mole (3,3 cm³) |
| Dichlorométhane | 100 cm³ |

### Mode opératoire :

Dans une fiole de 250cm³, dissoudre 4 g de 2-(7-méthoxy napht-1-yl) éthanol dans 100 cm³ de dichlorométhane et ajouter 3,3 cm³ de triéthylamine. Refroidir dans un bain de glace à -10°C, puis ajouter goutte à goutte et sous agitation 2,75 g de chlorure de mésyle. Laisser agiter pendant 30 minutes. Extraire la phase aqueuse par 3 fois 50 cm³ de dichlorométhane, laver la phase organique par 3 fois 20 cm³ d'une solution d'acide chlorhydrique 1N, puis à l'eau, la sécher sur du carbonate de potassium et l'évaporer. Recristalliser le résidu huileux.

| **Caractéristiques :** | |
|---|---|
| Masse molaire | 280,34 g/mol pour C₁₄H₁₆O₄S |
| Point de fusion | 62-63°C |
| Solvant de recristallisation | cyclohexane |
| Rendement | 94% |
| Solvant d'élution | Acétone-Toluène-Cyclohexane (4-4-2). |
| Rf | 0,72 |
| Aspect | poudre blanche |

| **Analyse élémentaire :** | | | |
|---|---|---|---|
| | **C%** | **H%** | **O%** |
| Calculé | 59,98 | 5,76 | 22,83 |
| Trouvé | 59,83 | 5,60 | 22,59 |

### Analyse spectroscopique dans l'infra-rouge:

- 3000-2820 cm⁻¹ : ν CH (alkyles)
- 1620 et 1590 cm⁻¹ : ν C=C (aromatiques)
- 1330 cm⁻¹ : ν SO₂ asymétrique
- 1240 cm⁻¹ : ν OCH₃
- 1150 cm⁻¹ : ν SO₂ symétrique

| **Analyse spectroscopique de RMN du proton (CDCl**_{**3**}**, δ, 80 MHz) :** | |
|---|---|
| 2,85 ppm (singulet, 3H) | Hd |
| 3,50 ppm (triplet, 2H) | H_{b} J_{b-c} = 8,00 Hz |
| 3,95 ppm (singulet, 3H) | Hₐ |
| 4,55 ppm (triplet, 2H) | Hc J_{c-b} = 8,00 Hz |
| 7,10-7,40 ppm (massif, 4H) | H_{2,3,6,8} |
| 7,60-7,80 ppm (massif, 2H) | H_{4,5} |

### STADE D : 3-(7-METHOXY NAPHT-1-YL)PROPIONITRILE

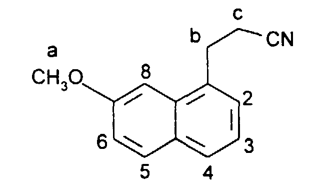

### 1) A partir du sel d'ammonium quaternaire

| **Réactifs :** | |
|---|---|
| Iodure de triméthyl 2-(7-méthoxy napht-1-yl)éthylammonium | 0,002 mole (0,74 g) |
| Cyanure de potassium | 0,006 mole (0,4 g) |
| Diméthylformamide | 10 cm³ |

### Mode opératoire :

Dans une fiole de 100 cm³, mettre 10 cm³ de diméthylformamide et 0,74 g de sel d'ammonium quaternaire. Chauffer à 75°C. Puis ajouter 0,4 g de cyanure de potassium. Laisser à 75°C pendant 2 heures. Laisser refroidir. Verser le milieu dans un mélange d'eau et de glace. Filtrer le précipité obtenu et le recristalliser.
Rendement : 10 % (produit brut)

### 2) A partir du mésylate

| **Réactifs :** | |
|---|---|
| Mésylate du 2-(7-méthoxy napht-1-yl) éthanol | 0,015 mole (4,2 g) |
| Cyanure de potassium | 0,045 mole (2,93 g) |
| Diméthylsulfoxyde | 20 cm³ |

### Mode opératoire :

Dans une fiole de 100 cm³, dissoudre 4,2 g de mésylate du 2-(7-méthoxy napht-1-yl) éthanol dans 20 cm³ de diméthylsulfoxyde et ajouter 2,93 g de cyanure de potassium. Mettre à reflux pendant 2 heures. Laisser refroidir, verser le milieu dans un mélange eau-glace. Filtrer le nitrile qui a précipité et le recristalliser.
Rendement : 95 %

| **Caractéristiques :** | |
|---|---|
| Masse molaire | 211,26 g/mol pour C₁₄H₁₃NO |
| Point de fusion | 62-64°C |
| Solvant de recristallisation | hexane |
| Solvant d'élution | Acétone-Toluène-Cyclohexane (4-4-2) |
| Rf | 0,80 |
| Aspect | poudre blanche |

| **Analyse élémentaire :** | | | |
|---|---|---|---|
| | **C%** | **H%** | **O%** |
| Calculé | 79,60 | 6,20 | 7,57 |
| Trouvé | 79,67 | 6,20 | 7,72 |

### Analyse spectroscopique dans l'infra-rouge :

- 3020-2820 cm⁻¹ : ν CH (alkyles)
- 2220 cm⁻¹ : ν CN (nitrile)
- 1610 et 1590 cm⁻¹ : ν C=C (aromatiques)
- 1240 cm⁻¹ : ν OCH₃

| **Analyse spectroscopique de RMN du proton (CDCl**_{**3**}**, δ, 80 MHz) :** | |
|---|---|
| 2,80 ppm (triplet, 2H) | H_{c} J_{b-c} = 7,70 Hz |
| 3,40 ppm (triplet, 2H) | H_{b} J_{b-c} = 7,70 Hz |
| 4,00 ppm (singulet, 3H) | Hₐ |
| 7,20-7,40 ppm (massif, 4H) | H_{2,3,6,8} |
| 7,70-7,90 ppm (massif, 2H) | H_{4,5} |

### STADE E : CHLORHYDRATE DE LA 3-(7-METHOXY NAPHT-1-YL)PROPYLAMINE

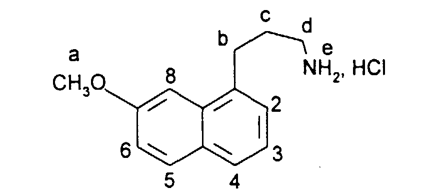

| **Réactifs :** | |
|---|---|
| 3-(7-méthoxy napht-1-yl)propionitrile | 0,006 mole (1,51 g) |
| Nickel de Raney | 1 g |
| Ethanol | 150 cm³ |

### Mode opératoire :

Dans un autoclave, verser 1,51 g du 3-(7-méthoxy napht-1-yl) propionitrile préalablement dissous dans 150 cm³ d'éthanol, ajouter 1 g de nickel de Raney. Saturer la solution en ammoniac. Mettre sous pression d'hydrogène (50 bars) et à 60°C. Laisser agiter pendant 12 heures. Laisser refroidir à température ambiante. Filtrer le milieu et évaporer le solvant. Redissoudre dans l'éther anhydre et ajouter de l'éther anhydre saturé en HCI. Essorer le chlorydrate obtenu puis le recristalliser.

| **Caractéristiques :** | |
|---|---|
| Masse molaire | 251,75 g/mol pour C₁₄H₁₈NOCl |
| Point de fusion | 197-200°C |
| Solvant de recristallisation | cyclohexane-éthanol (1-1) |
| Rendement | 91 % |
| Solvant d'élution | Acétone-Toluène-Cyclohexane (4-4-2) |
| Rf | 0,05 |
| Aspect | solide blanc |

| **Analyse élémentaire :** | | | | |
|---|---|---|---|---|
| | **C%** | **H%** | **O%** | **N%** |
| Calculé | 66,79 | 7,21 | 6,36 | 5,56 |
| Trouvé | 66,79 | 7,22 | 6,35 | 5,62 |

### Analyse spectroscopique dans l'infra-rouge:

- 3100-2640 cm⁻¹ : ν NH₃⁺, large pic
- 1620 et 1590 cm⁻¹ : ν C=C (aromatiques)
- 1250 cm⁻¹ : ν OCH₃

| **Analyse spectroscopique de RMN du proton (DMSO d**_{**6**}**, δ, 300 MHz)** | |
|---|---|
| 2,00 ppm (multiplet, 2H) | H_{c} |
| 2,90 ppm (triplet, 2H) | H_{b} J_{b-c} = 7,72 Hz |
| 3,10 ppm (triplet, 2H) | H_{d} J_{d-c} = 7,27 Hz |
| 3,95 ppm (singulet, 3H) | Hₐ |
| 7,15-7,40 ppm (massif, 4H) | H_{2,3,6,8} |
| 7,74 ppm (doublet, 1H) | H₄ Jₒᵣₜₕₒ = 7,78 Hz |
| 7,85 ppm (doublet, 1H) | H₅ Jₒᵣₜₕₒ = 8,94 Hz |
| 8,12 ppm (singulet, 3H) | NH₃⁺, échangeables dans D₂O |

### PREPARATION 2: 3-(7-ETHYL-1,2,3,4-TETRAHYDRONAPHT-1-YL)PROPYLAMINE

### STADE A : ACIDE-4-OXO-4-(4-ETHYL-PHENYL)BUTYRIQUE

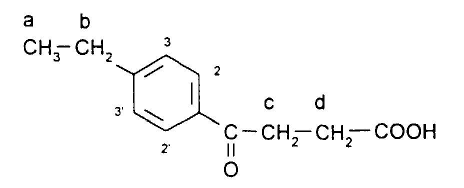

| **Réactifs :** | |
|---|---|
| Ethyl benzène | 0,05 mole (5 cm3) |
| Chlorure d'aluminium | 0,02 mole (2,6 g) |
| Anhydride succinique | 0,01 mole (1 g) |

### Mode opératoire :

Dans une fiole de 50 cm³, mélanger sous agitation magnétique 5 cm³ d'éthyl benzène et 2,6 g de chlorure d'aluminium. Refroidir la solution dans un bain de glace puis ajouter 1 g d'anhydride succinique. Agiter 1 h 30 à la température de 0°C puis 3 h à température ambiante. Verser le mélange réactionnel dans la glace. Acidifier par addition d'acide chlorhydrique 1N (pH 3-4). Extraire par 3 volumes d'éther. Les phases organiques sont lavées 3 fois par une solution de carbonate de potassium à 10 %. Les phases aqueuses sont rassemblées et acidifiées par addition d'acide chlorhydrique concentré. Le précipité obtenu est essoré puis recristallisé.

| **Caractéristiques :** | |
|---|---|
| Masse molaire | 206,23 g/mole pour C₁₂H₁₄O₃ |
| Aspect | poudre blanche |
| Solvant de recristallisation | cyclohexane |
| Rendement | 57 % |
| Température de fusion | 106-108°C |
| Rf | 0,36 |
| Eluant | Acétone-Toluène-Cyclohexane (2-2-1) |

| **Analyse spectroscopique dans l'infrarouge :** | | |
|---|---|---|
| 2960-2920 | cm⁻¹ | υ CH alkyles |
| 1710 | cm⁻¹ | υ CO cétone |
| 1670 | cm⁻¹ | υ CO acide |
| 1600 | cm⁻¹ | υ C=C aromatiques |

| **Analyse spectroscopique de RMN du proton (80 MHz, DMSO d**_{**6**}**, δ) :** | | |
|---|---|---|
| 1,2 ppm (triplet, 3H) | CH₃ (a) | J_{a-b} = 6,60 Hz |
| 2,6 ppm (multiplet, 4H) | CH₂ (b) et CH₂ (d) | Jb-a = Jd-c = 6,60 Hz |
| 3,2 ppm (triplet, 2H) | CH₂ (c) | Jc-d = 6,60 Hz |
| 7,4 ppm (doublet, 2H) | H₃ et H₅ | Jₒᵣₜₕₒ = 8,80 Hz |
| 7,9 ppm (doublet, 2H) | H₂ et H₆ | Jₒᵣₜₕₒ = 8,80 Hz |
| 12,1 ppm (multiplet, 1H) | COOH | |

| **Analyse spectrométrique de masse :** | | |
|---|---|---|
| m/e | 206 | M⁺ |
| m/e | 207 | (M + 1)⁺ |

### STADE B : ACIDE 4-(4-ETHYL-PHENYL)BUTYRIQUE

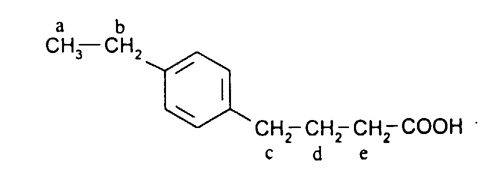

| **Réactifs :** | |
|---|---|
| Acide-4-oxo-4-(4-éthyl-phényl) butyrique (stade A) | 0,012 mole (2,5 g) |
| Triéthylsilane | 0,028 mole (3,2 g) |
| Acide trifluoroacétique | 0,12 mole (19 cm³) |

### Mode opératoire :

Dans une fiole de 100 cm³, 2,5 g d'acide-4-oxo-4-(4-éthyl-phényl) butyrique sont dissous sous agitation magnétique dans 19 cm³ d'acide trifluoroacétique. Ajouter goutte à goutte 3,2 g de triéthylsilane. Agiter 86 heures à température ambiante. Le mélange réactionnel est versé dans la glace. Extraire par 3 volumes d'éther. Les phases organiques sont lavées 3 fois par une solution de carbonate de potassium à 10 %. Les phases aqueuses sont rassemblées puis acidifiées par addition d'acide chlorhydrique concentré, jusqu'à pH 3-4. Le précipité obtenu est essoré puis recristallisé.

| **Caractéristiques :** | |
|---|---|
| Masse molaire | 192,25 g/mole pour C₁₂H₁₆O₂ |
| Aspect | poudre blanche |
| Solvant de recristallisation | eau |
| Rendement | 65 % |
| Température de fusion | 71-73°C |
| Rf | 0,67 |
| Eluant | Acétone-Toluène-Cyclohexane (2-2-1) |

| **Analyse spectroscopique dans l'infrarouge :** | | |
|---|---|---|
| 3280-2780 | cm⁻¹ | υ OH acide |
| 2940-2850 | cm⁻¹ | υ CH alkyles |
| 1680 | cm⁻¹ | υ CO acide |
| 1510 | cm⁻¹ | υ C=C aromatiques |

| **Analyse spectroscopique de RMN du proton (300 MHz, DMSO d**_{**6**}**, δ) :** | | |
|---|---|---|
| 1,14 ppm (triplet, 3H) | CH₃ (a) | J_{a-b} = 7,63 Hz |
| 1,76 ppm (multiplet, 2H) | CH₂ (d) | |
| 2,20 ppm (triplet, 2H) | CH₂ (e) | J_{d-e} = 7,65 Hz |
| 2,55 ppm (multiplet, 4H) | CH₂ (c) et CH₂ (b) | |
| 7,11 ppm (multiplet, 6H) | H aromatiques | |
| OH acide non observé | | |

| **Analyse spectrométrique de masse :** | | |
|---|---|---|
| m/e | 192 | M⁺ |
| m/e | 193 | (M + 1)⁺ |

### STADE C : 7-ETHYL TETRALONE

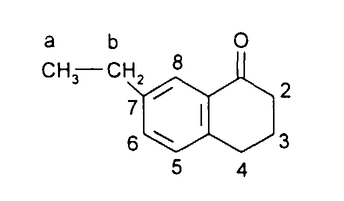

| **Réactifs :** | |
|---|---|
| Acide-4-(4-éthyl phényl) butyrique (stade B) | 0,013 mole (2,5 g) |
| Acide polyphosphorique | 25 g |

### Mode opératoire :

Dans un ballon à col rodé de 100 cm³, verser 25 g d'acide polyphosphorique. Ajouter 2,5 g d'acide 4-(4-éthyl phényl) butyrique. Agiter 6 h à une température de 45°C. Le mélange réactionnel est versé dans la glace. Extraire par 3 volumes d'éther. Les phases organiques sont lavées 3 fois par une solution de carbonate de potassium à 10 %, séchées sur sulfate de magnésium puis évaporées à sec.
L'huile obtenue est purifiée par chromatographie sur colonne.

| **Caractéristiques :** | |
|---|---|
| Masse molaire | 174,23 g/mole pour C₁₂H₁₄O₂ |
| Aspect | huile incolore |
| Rf | 0,35 |
| Eluant | toluène-cyclohexane (1-2) |
| Rendement | 55 % |

| **Analyse spectroscopique dans l'infrarouge :** | | |
|---|---|---|
| 3010 | cm⁻¹ | ν CH aromatiques |
| 2980-2860 | cm⁻¹ | ν CH alkyles |
| 1680 | cm⁻¹ | ν CO cétone |
| 1605 | cm⁻¹ | ν C=C aromatiques |

| **Analyse spectroscopique de RMN du proton (300 MHz, DMSO d**_{**6**}**, δ) :** | |
|---|---|
| 1,13 ppm (triplet, 3H) | CH₃ (a), J_{a-b} = 7,68 Hz |
| 2,01 ppm (multiplet, 2H) | CH₂ (3) |
| 2,59 ppm (multiplet, 4H) | CH₂ (b) et CH₂ (4) |
| 2,88 ppm (triplet, 2H) | CH₂ (2), J₂₋₃ = 5,77 Hz |
| 7,25 ppm (doublet, 1H) | H₅, Jₒᵣₜₕₒ = 8,59 Hz |
| 7,39 ppm (doublet dédoublé, 1H) | H₆, Jₒᵣₜₕₒ = 8,59 Hz, J_{méta} = 2,14 Hz |
| 7,70 ppm (doublet, 1H) | H₈, J_{méta} = 2,14 Hz |

| **Analyse spectrométrique de masse :** | |
|---|---|
| m/e 174 | M⁺ |
| m/e 175 | (M+1)⁺ |

### STADE D: 3-(7-ETHYL-1 ,2,3,4-TETRAHYDRO-NAPHT-1 -YL)PROPYLAMINE

Le composé obtenu au stade précédent est ensuite mis en réaction avec un réactif cyané afin d'obtenir le 3-(7-éthyl-1,2,3,4-tétrahydronapht-1-yl)propionitrile qui est ensuite hydrogéné, en procédant de façon analogue au stade D de la préparation 1, pour obtenir le composé du titre.

### PREPARATION 3 : 3-(5-ETHYL-BENZO[b]THIOPHEN-3-YL)PROPYLAMINE

### PREPARATION 4 : 3-(7-METHYL-1,2,3,4-TETRAHYDRO-NAPHT-1-YL)PROPYLAMINE

En procédant comme dans la préparation 2 mais en partant du toluène au lieu de l'éthylbenzène, on obtient le composé du titre.

### PREPARATION 5 : 3-(7-PROPYL-1,2,3,4-TETRAHYDRO-NAPHT-1-YL)PROPYLAMINE

En procédant comme dans la préparation 2, mais en remplaçant l'éthylbenzène par le propylbenzène, on obtient le composé du titre.

### PREPARATION 6 : 3-(7-BUTYL-1,2,3,4-TETRAHYDRO-NAPHT-1-YL)PROPYLAMINE

En procédant comme dans la préparation 2, mais en remplaçant l'éthylbenzène par le butylbenzène, on obtient le composé du titre.

### PREPARATION 7 : 3-(5-METHYL-BENZOFURAN-3-YL)PROPYLAMINE

### PREPARATION 8 : 3-(5-ETHYL-BENZOFURAN-3-YL)PROPYLAMINE

### PREPARATION 9 : 3-(7-METHOXY-1,2,3,4-TETRAHYDRONAPHT-1-YL) PROPYLAMINE

### PREPARATION 10 : 3-(5-METHOXY-BENZOFURAN-3-YL)PROPYLAMINE

### PREPARATION 11 : 3-(NAPHT-1-YL)PROPYLAMINE

### PREPARATION 12 : 3-(5-METHOXY-BENZOTHIOPHEN-3-YL)PROPYLAMINE

### PRFPARATION 13 : 3-(5-METHOXY-INDOL-3-YL)PROPYLAMINE Pol. J. Pharmacol. Pharm. 1979, 31(2), pp 149-156

### PREPARATION 14 : 3-(5-METHYL-BENZOTHIOPHEN-3-YL)PROPYLAMINE Monatsch. Chem. 1968, 99(5), pp. 2095-9

### PRFPARATION 15 : 4-(7-METHOXY NAPHT-1-YL)BUTYLAMINE

### Stade A : Acide 3-(7-méthoxy napht-1-yl)propanoïque

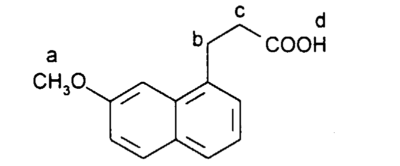

| **Réactifs :** | |
|---|---|
| 3-(7-méthoxy napht-1-yl)propionitrile | 1 g (4,7 x 10⁻³ mole) |
| Solution aqueuse de soude 6N | 10 cm³ (6 x 10⁻² mole) |
| Méthanol | 10 cm³ |

### Mode opératoire :

Dissoudre le 3-(7-méthoxy napht-1-yl) propionitrile dans 10 cm³ de méthanol dans une fiole de 100 cm³. Ajouter la solution aqueuse de soude et porter le milieu à reflux pendant une nuit. Laisser refroidir et acidifier le milieu par une solution aqueuse de HCI 6N puis filtrer le précipité formé. Le recristalliser.

| **Caractéristiques :** | |
|---|---|
| Masse molaire | 230,26 g pour C₁₄H₁₄O₃ |
| Aspect | solide blanc |
| Température de fusion | 154-155°C |
| Rf | 0,40 |
| Eluant | Acétone/Toluène/Cyclohexane (ci-après ATC) (4/4/2) |
| Rendement | 90 % |
| Solvant de recristallisation | toluène |

| **Analyse élémentaire :** | | | |
|---|---|---|---|
| | **C%** | **H%** | **O%** |
| Calculé | 73,03 | 6,13 | 20,85 |
| Trouvé | 73,14 | 6,20 | 20,65 |

| **Analyse spectroscopique dans l'infrarouge :** | | |
|---|---|---|
| 1700 | cm⁻¹ | υ CO acide |
| 1620 et 1600 | cm⁻¹ | υ C=C |
| 1260 | cm⁻¹ | υ CH₃O |

| **Analyse spectroscopique de RMN (80 MHz, DMSO, δ) :** | | |
|---|---|---|
| 2,65 ppm (triplet, 2H) | H_{b} | J_{b-c} = 7,60 Hz |
| 3,30 ppm (triplet, 2H) | H_{c} | Jc-b = 7,60 Hz |
| 3,90 ppm (singulet, 3H) | Hₐ | |
| 7,10-7,40 ppm (massif, 4H) | H2,3,6,8 | |
| 7,60-7,95 ppm (massif, 2H) | H4,5 | |
| 12,20 ppm (signal, 1H) | H_{d} échangeable dans D₂O | |

### STADE B : 3-(7-METHOXY-NAPHT-1-YL)PROPANOATE DE METHYLE

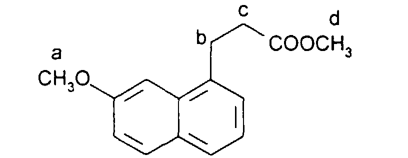

| **Réactifs :** | |
|---|---|
| Acide 3-(7-méthoxy napht-1-yl)propanoique | 0,2 g (0,9 x 10⁻³ mole) |
| Chlorure de thionyle | 0,25 cm³ (3,5 x 10⁻³ mole) |
| Méthanol | 20 cm³ |

### Mode opératoire :

Dans une fiole de 100 cm³ placé dans un bain de glace à - 5°C, dissoudre l'acide dans le méthanol. Ajouter goutte à goutte le chlorure de thionyle et laisser sous agitation pendant 1 h. Evaporer et reprendre le résidu par 10 cm³ d'éther. Laver la phase organique par une solution aqueuse de carbonate de potassium à 10 % et par de l'eau. Sécher sur CaCl₂, filtrer et concentrer à l'évaporateur rotatif.

| **Caractéristiques** : | |
|---|---|
| Masse molaire | 244,29 g pour C₁₅H₁₆O₃ |
| Aspect | huile |
| Rf | 0,67 |
| Eluant | ATC (4/4/2) |
| Rendement | 89 % |

| **Analyse élémentaire :** | | | |
|---|---|---|---|
| | **C%** | **H%** | **O%** |
| Calculé | 73,75 | 6,60 | 19,65 |
| Trouvé | 73,41 | 6,63 | 19,47 |

| **Analyse spectroscopique dans l'infrarouge :** | | |
|---|---|---|
| 3020 - 2800 | cm⁻¹ | υ CH |
| 1730 | cm⁻¹ | υ CO ester |
| 1620 et 1590 | cm⁻¹ | υ C=C |
| 1250 | cm⁻¹ | υ CH₃O |

| **Analyse spectroscopique de RMN (300 MHz,CDCl**_{**3**}**, δ) :** | | |
|---|---|---|
| 2,75 ppm (triplet, 2H) | H_{b} | J_{b-c} = 7,95 Hz |
| 3,34 ppm (triplet, 2H) | H_{c} | J_{c-b} = 7,94 Hz |
| 3,68 ppm (singulet, 3H) | H_{d} | |
| 3,90 ppm (singulet, 3H) | Hₐ | |
| 7,13-7,30 ppm (massif, 4H) | H_{2,3,6,8} | |
| 7,63 ppm (doublet, 1H) | H₄ | Jₒᵣₜₕₒ = 7,77 Hz |
| 7,73 ppm (doublet, 1H) | H₅ | Jₒᵣₜₕₒ = 8,95 Hz |

### STADE C : 3-(7-METHOXY NAPHT-1-YL)-PROPANOL

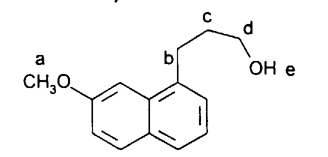

| **Réactifs :** | |
|---|---|
| 3-(7-méthoxy napht-1-yl)-propanoate de méthyle | 0,5 g (2 x 10⁻³ mole) |
| LiAlH₄ | 0,3 g (8 x 10⁻³ mole) |
| Ether anhydre | 10 cm³ |

### Mode opératoire :

Dans une fiole de 50 cm³ placé dans un bain de glace à - 5°C, mettre LiAlH₄ et l'éther puis ajouter goutte à goutte l'ester préalablement dilué dans de l'éther. Laisser agiter pendant 1h et hydrolyser le milieu en le versant sur de l'eau glacée. Filtrer le milieu et l'extraire par de l'éther. Sécher la phase organique, la filtrer et l'évaporer.

| **Caractéristiques :** | |
|---|---|
| Masse molaire | 217,29 g pour C₁₄H₁₆O₂ |
| Aspect | solide blanc |
| Température de fusion | 38-39°C |
| Rf | 0,13 |
| Eluant | chloroforme |
| Rendement | 88 % |
| Solvant de recristallisation | cyclohexane |

| **Analyse élémentaire :** | | | |
|---|---|---|---|
| | **C%** | **H%** | **O%** |
| Calculé | 77,39 | 7,42 | 14,73 |
| Trouvé | 77,35 | 7,49 | 14,68 |

| **Analyse spectroscopique dans l'infrarouge :** | | |
|---|---|---|
| 3300 | cm⁻¹ | υ OH large bande |
| 3040-2800 | cm⁻¹ | υ CH |
| 1620 et 1590 | cm⁻¹ | υ C=C |
| 1250 | cm⁻¹ | υ CH₃O |

| **Analyse spectroscopique de RMN (300 MHz,CDCl**_{**3**}**, δ) :** | | | |
|---|---|---|---|
| 2,00-2,10 | ppm (multiplet, 2H) | H_{c} | |
| 3,15 | ppm (triplet, 2H) | H_{b} | J_{b-c} = 7,59 Hz |
| 3,75 | ppm (triplet, 2H) | H_{d} | J_{d-c} = 6,24 Hz |
| 3,95 | ppm (singulet, 3H) | Hₐ | |
| 7,13-7,37 | ppm (massif, 4H) | H_{2,3,6,8} | |
| 7,66 | ppm (doublet, 1H) | H₄ | Jₒᵣₜₕₒ = 7,83 Hz |
| 7,76 | ppm (doublet, 1H) | H₅ | Jₒᵣₜₕₒ = 8,92 Hz |

### STADE D : MESYLATE DU 3-(7-METHOXY NAPHT-1-YL)PROPAN-1-OL

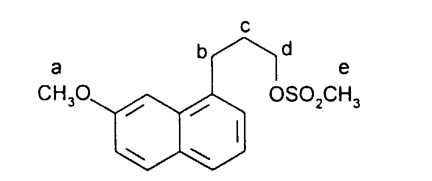

| **Réactifs :** | |
|---|---|
| 3-(7-méthoxy-napht-1-yl)propanol | 5,1 g (23,5 x 10⁻³ mole) |
| CH₃SO₂Cl | 3,3 g (28,2 x 10⁻³ mole) |
| NEt₃ | 3,9 cm³ (28,2 x 10⁻³ mole) |
| CH₂Cl₂ | 100 cm³ |

### Mode opératoire :

Dans un ballon de 250 cm3, dissoudre le 3-(7-methoxy-napht-1-yl)propanol dans le CH₂Cl₂. Ajouter la triéthylamine. Placer le ballon dans un bain de glace et de sel (-5°C). Ajouter goutte à goutte le chlorure de mésyle. Laisser sous agitation pendant 2 h. La phase organique est lavée par 3 x 20 cm³ HCI puis par de l'eau jusqu'a neutralité des eaux. Sécher sur CaCl₂, filtrer et concentrer à l'évaporateur rotatif. Recristalliser le solide obtenu.

| **Caractéristiques :** | |
|---|---|
| Masse molaire | 294,37 g pour C₁₅H₁₈O₄S |
| Température de fusion | 52-54°C |
| Rf | 0,71 |
| Eluant | chloroforme - méthanol (9-1) |
| Rendement | 71 % |
| Solvant de recristallisation | toluène / cyclohexane (1/3) |

| **Analyse élémentaire :** | | | |
|---|---|---|---|
| | **C%** | **H%** | **O%** |
| Calculé | 61,20 | 6,16 | 21,74 |
| Trouvé | 61,29 | 6,12 | 21,68 |

| **Analyse spectroscopique dans l'infrarouge :** | | |
|---|---|---|
| 3040-2820 | cm⁻¹ | ν CH alkyles |
| 1610 et 1590 | cm⁻¹ | v C=C |
| 1330 | cm⁻¹ | ν SO₂ asym |
| 1260 | cm⁻¹ | ν OCH₃ |
| 1170 | cm⁻¹ | ν SO₂ sym |

| **Analyse spectroscopique de RMN (300 MH**_{**z**}**, DMSO, δ) :** | | | |
|---|---|---|---|
| 2,08 | ppm (multiplet, 2H) | H_{c} | |
| 3,12 | ppm (triplet, 2H) | H_{b} | J_{b-c} = 7,75 Hz |
| 3,22 | ppm (singulet, 3H) | Hₑ | |
| 3,92 | ppm (singulet, 3H) | Hₐ | |
| 4,32 | ppm (triplet, 2H) | H_{d} | J_{d-c} = 6,20 Hz |
| 7,19 | ppm (doublet dédoublé, 1H) | H₆ | Jₒᵣₜₕₒ = 8,93 Hz |
| | | | Jₘₑₜₐ = 2,38 Hz |
| 7,26-7,37 | ppm (massif, 3H) | H_{2,3,8} | |
| 7,72 | ppm (doublet, 1H) | H₄ | J = 7,88 Hz |
| 7,85 | ppm (doublet, 1H) | H₅ | J = 8,90 Hz |

### STADE E : 4-(7-METHOXY-NAPHT-1-YL)BUTYRONITRILE

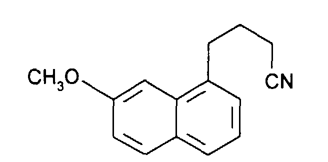

| **Réactifs :** | |
|---|---|
| Mésylate du 3-(7-méthoxy napht-1-yl)propan-1-ol | 3 g (10,2 x 10⁻³ mole) |
| KCN | 2 g (30,6 x 10⁻³ mole) |
| DMSO | 20 cm³ |

### Mode opératoire :

Dans une fiole de 100 cm³, dissoudre le mésylate du 3-(7-méthoxy napht-1-yl)propan-1-ol, obtenu au stade D, dans du DMSO. Ajouter KCN et porter à reflux pendant 2 h. Laisser refroidir. Verser dans un mélange eau / glace. Extraire à l'éther. Laver la phase organique avec de l'eau, la sécher sur CaCl₂ et l'évaporer. Recristalliser le produit obtenu.

| **Caractéristiques :** | |
|---|---|
| Masse molaire | 225,29 g pour C₁₅H₁₅NO |
| Température de fusion | 52-54°C |
| Rf | 0,39 |
| Eluant | ATC (2-3-5) |
| Rendement | 90 % |
| Solvant de recristallisation | alcool /eau (1/5) |

| **Analyse élémentaire :** | | | | |
|---|---|---|---|---|
| | **C%** | **H%** | **O%** | **N%** |
| Calculé | 79,97 | 6,71 | 7,10 | 6,22 |
| Trouvé | 79,58 | 6,78 | 7,49 | 6,15 |

| **Analyse spectroscopique dans l'infrarouge :** | | |
|---|---|---|
| 3040-2820 | cm⁻¹ | ν CH alkyles |
| 2240 | cm⁻¹ | ν CN |
| 1620 et 1590 | cm⁻¹ | ν C=C |
| 1250 | cm⁻¹ | ν OCH₃ |

| **Analyse spectroscopique de RMN (300 MH**_{**z**}**, CDCl**_{**3**}**, δ) :** | | | |
|---|---|---|---|
| 2,09 | ppm (multiplet, 2H) | H_{c} | |
| 2,35 | ppm (triplet, 2H) | H_{d} | J_{d-c} = 6,87 Hz |
| 3,18 | ppm (triplet, 2H) | H_{b} | J_{b-c} = 7,44 Hz |
| 3,94 | ppm (singulet, 3H) | Hₐ | |
| 7,16 | ppm (doublet dédoublé, 1H) | H₆ | Jₒᵣₜₕₒ = 8,91 Hz |
| | | | J_{méta} = 2,44 Hz |
| 7,23-7,30 | ppm (massif, 3H) | H_{2,3,8} | |
| 7,67 | ppm (doublet dédoublé, 1H) | H₄ | Jₒᵣₜₕₒ = 7,31 Hz |
| | | | J_{méta} = 1,77 Hz |
| 7,76 | ppm (doublet, 1H) | H₅ | Jortho = 8,91 Hz |

### STADE F : 3-(7-METHOXY-NAPHT-1-YL)BUTYLAMINE

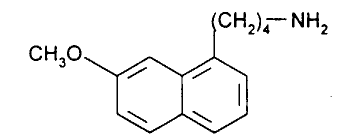

Le composé obtenu au stade E est hydrogéné en présence de Nickel de Raney afin d'obtenir le produit du titre.

### PREPARATION 16 : CHLORHYDRATE DE N-METHYL N-[3-(7-METHOXY NAPHT-1-YL)PROPYL]AMINE

Le chlorhydrate de N-[3-(7-méthoxy napht-1-yl)propyl] amine obtenu à la préparation 1 est méthylé pour conduire à la formation de chlorhydrate de N-méthyl N-[3-(7-méthoxy napth-1-yl)propyl)amine.

### PREPARATION 17 : 3-(7-ETHOXY NAPHT-1-YL)PROPYLAMINE

En procédant comme dans la préparation 1 mais en utilisant au départ l'acide (7-éthoxy napht-1-yl)acétique, on obtient le composé du titre.

### PREPARATION 18 : 3-(7-PROPOXY NAPHT-1-YL)PROPYLAMINE

En procédant comme dans la préparation 1 mais en utilisant au départ l'acide (7-propoxy napht-1-yl)acétique, on obtient le composé du titre.

### PREPARATION 19 : 3-(7-BUTYLOXY NAPHT-1-YL)PROPYLAMINE

En procédant comme dans la préparation 1 mais en utilisant au départ l'acide (7-butyloxy napht-1-yl)acétique, on obtient le composé du titre.

### PREPARATION 20 : 3-(7-PENTOXY NAPHT-1-YL)PROPYLAMINE

En procédant comme dans la préparation 1 mais en utilisant au départ l'acide (7-pentoxy napht-1-yl)acétique, on obtient le composé du titre.

### PREPARATION 21 : 3-(7-HEXYLOXY NAPHT-1-YL)PROPYLAMINE

En procédant comme dans la préparation 1 mais en utilisant au départ l'acide (7-hexyloxy napht-1-yl)acétique, on obtient le composé du titre.

### PREPARATION 22 : 3-(7-CYCLOHEXYLOXY NAPHT-1-YL)PROPYLAMINE

En procédant comme dans la préparation 1 mais en utilisant au départ l'acide (7-cyclohexyloxy napht-1-yl)acétique, on obtient le composé du titre.

### PREPARATION 23 : 3-(7-CYCLOPROPYLMETHOXY NAPHT-1-YL)PROPYLAMINE

En procédant comme dans la préparation 1 mais en utilisant au départ l'acide (7-cyclopropylméthoxy napht-1-yl)acétique, on obtient le composé du titre.

### PREPARATION 24 : 3-(7-METHYL NAPHT-1-YL)PROPYLAMINE

En procédant comme dans la préparation 1 mais en utilisant au départ l'acide (7-méthyl napht-1-yl)acétique, on obtient le composé du titre.

### PREPARATION 25 : 3-(7-ETHYL NAPHT-1-YL)PROPYLAMINE

En procédant comme dans la préparation 1 mais en utilisant au départ l'acide (7-éthyl napht-1-yl)acétique, on obtient le composé du titre.

### PREPARATION 26 : 3-(7-PROPYL NAPHT-1-YL)PROPYLAMINE

En procédant comme dans la préparation 1 mais en utilisant au départ l'acide (7-propyl napht-1-yl)acétique, on obtient le composé du titre.

### PREPARATION 27 : 3-(7-BUTYL NAPHT-1-YL)PROPYLAMINE

En procédant comme dans la préparation 1 mais en utilisant au départ l'acide (7-butyl napht-1-yl)acétique, on obtient le composé du titre.

### PREPARATION 28 : 3-(7-PENTYL NAPHT-1-YL)PROPYLAMINE

En procédant comme dans la préparation 1 mais en utilisant au départ l'acide (7-pentyl napht-1-yl)acétique, on obtient le composé du titre.

### PREPARATION 29 : 3-(7-HEXYL NAPHT-1-YL)PROPYLAMINE

En procédant comme dans la préparation 1 mais en utilisant au départ l'acide (7-hexyl napht-1-yl)acétique, on obtient le composé du titre.

### PREPARATION 30 : 6-(7-METHOXY NAPHT-1-YL)HEXYLAMINE

### EXEMPLE 1 : N-[3-(7-METHOXY NAPHT-1-YL)PROPYL]ACETAMIDE

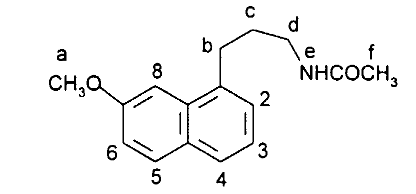

| **Réactifs :** | |
|---|---|
| Chlorhydrate de la 3-(7-méthoxy napht-1-yl)propylamine | 0,005 mole (1,26 g) |
| (préparation 1) | |
| Carbonate de potassium | 0,015 mole |
| Chlorure d'acétyle | 0,010 mole |
| Chloroforme | 50 cm³ |

### Mode opératoire :

Après avoir dissous 0,005 mole (soit 1,26 g) de chlorhydrate de la 3-(7-méthoxy napth-1-yl)propylamine dans 20 cm³ d'eau, ajouter 0,015 mole de carbonate de potassium. L'amine précipite . Ensuite, ajouter 50 cm³ de chloroforme et, goutte à. goutte, 0,010 mole de chlorure d'acétyle. Laisser sous agitation pendant 2 heures. Acidifier le milieu par l'acide chlorydrique 2N, puis agiter 15 minutes. Extraire la phase aqueuse acide par 3 fois 50 cm³ de chloroforme. Laver les phases organiques par de l'eau, les sécher sur du chlorure de calcium et les évaporer. La purification des produits s'effectue soit par recristallisation soit par chromatographie sur colonne.

| **Caractéristiques :** | |
|---|---|
| Masse molaire | 257,33 g/mole pour C₁₆H₁₉NO₂ |
| Point de fusion | 66-68°C |
| Solvant de recristallisation | cyclohexane |
| Rendement | 56 % |
| Solvant d'élution | Acétone-Toluène-Cyclohexane (4-4-2) |
| Rf | 0,25 |

| **Analyse élémentaire :** | | | | |
|---|---|---|---|---|
| | **C%** | **H%** | **O%** | **N%** |
| Calculé | 74,68 | 7,44 | 12,43 | 5,44 |
| Trouvé | 74,83 | 7,61 | 12,21 | 5,26 |

| **Analyse spectroscopique dans l'infrarouge :** | | |
|---|---|---|
| 3300 | cm⁻¹ | ν NH (amides) |
| 3020-2800 | cm⁻¹ | ν CH (alkyles) |
| 1630 | cm⁻¹ | ν CO (amide) |
| 1590 | cm⁻¹ | ν C=C (aromatiques) |
| 1250 | cm⁻¹ | ν OCH₃ |

| **Analyse spectroscopique de RMN du proton (DMSO d**_{**6**}**, δ, 300 MHz) :** | | | |
|---|---|---|---|
| 1,70 | ppm (multiplet, 2H) | H_{c} | |
| 1,83 | ppm (singulet, 3H) | H_{f} | |
| 3,00 | ppm (triplet, 2H) | H_{b} | J_{b-c} = 7,70 Hz |
| 3,14 | ppm (triplet, 2H) | H_{d} | J_{d-c} = 7,27 Hz |
| 3,91 | ppm (singulet, 3H) | Hₐ | |
| 7,14-7,36 | ppm (massif, 4H) | H_{2,3,6,8} | |
| 7,70 | ppm (doublet, 1H) | H₄ | Jₒᵣₜₕₒ = 7,83 Hz |
| 7,84 | ppm (doublet, 1H) | H₅ | Jₒᵣₜₕₒ = 8,95 Hz |
| 7,96 | ppm (signal, 1H) | Hₑ | |

### EXEMPLE 2 : N-[3-(7-METHOXY NAPHT-1-YL)PROPYL]CYCLOPROPYL CARBOXAMIDE

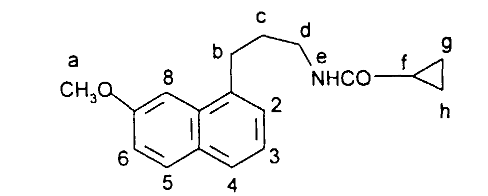

En procédant comme dans l'exemple 1 mais en remplaçant le chlorure d'acétyle par le chlorure de l'acide cyclopropanecarboxylique, on obtient le composé du titre.

| **Caractéristiques :** | |
|---|---|
| Masse molaire | 283,37 g/mole pour C₁₈H₂₁NO₂ |
| Point de fusion | 121-123°C |
| Solvant de recristallisation | cyclohexane |
| Rendement | 92 % |
| Solvant d'élution | Acétone-Toluène-Cyclohexane (4-4-2) |
| Rf | 0,59 |

| **Analyse élémentaire :** | | | | |
|---|---|---|---|---|
| | **C%** | **H%** | **O%** | **N%** |
| Calculé | 76,30 | 7,47 | 11,29 | 4,94 |
| Trouvé | 76,25 | 7,93 | 11,19 | 4,85 |

| **Analyse spectroscopique dans l'infrarouge :** | | |
|---|---|---|
| 3280 | cm⁻¹ | ν NH (amide) |
| 3050-2800 | cm⁻¹ | ν CH (alkyles) |
| 1620 | cm⁻¹ | ν CO (amide) |
| 1590 | cm⁻¹ | ν C=C (aromatiques) |
| 1250 | cm⁻¹ | ν OCH₃ |

| **Analyse spectroscopique de RMN du proton (DMSO d**_{**6**}**, δ, 300 MHz) :** | | | |
|---|---|---|---|
| 0,58-0,70 | ppm (massif, 4H) | H_{g,h} | |
| 1,55 | ppm (multiplet, 1H) | H_{f} | |
| 1,81 | ppm (multiplet, 2H) | H_{c} | |
| 3,00 | ppm (triplet, 2H) | H_{b} | jb-c = 7,67 Hz |
| 3,16 | ppm (multiplet, 2H) | H_{d} | |
| 3,93 | ppm (singulet, 3H) | Hₐ | |
| 7,14-7,36 | ppm (massif, 4H) | H_{2,3,6,8} | |
| 7,70 | ppm (doublet, 1H) | H₄ | Jortho = 7,90 Hz |
| 7,84 | ppm (doublet, 1H) | H₅ | Jortho = 8,95 Hz |
| 8,19 | ppm (signal, 1H) | Hₑ | |

### EXEMPLE 3 : N-[3-(7-METHOXYNAPHT-1-YL)PROPYL]PENTANAMIDE

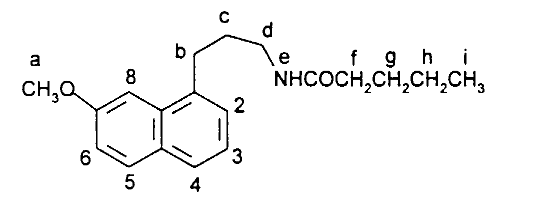

En procédant comme dans l'exemple 1 mais en remplaçant le chlorure d'acétyle par le chlorure de pentanoyle, on obtient le composé du titre.

| **Caractéristiques :** | |
|---|---|
| Masse molaire | 299,41 g/mole pour C₁₉H₂₅NO₂ |
| Point de fusion | 75-76°C |
| Solvant de recristallisation | cyclohexane |
| Rendement | 56 % |
| Solvant d'élution | Acétone-Toluène-Cyclohexane (4-4-2) |
| Rf | 0,57 |

| **Analyse élémentaire :** | | | | |
|---|---|---|---|---|
| | **C%** | **H%** | **O%** | **N%** |
| Calculé | 76,22 | 8,42 | 10,69 | 4,68 |
| Trouvé | 75,90 | 8,81 | 10,59 | 4,57 |

| **Analyse spectroscopique dans l'infrarouge :** | | |
|---|---|---|
| 3280 | cm⁻¹ | ν NH (amide) |
| 3040-2800 | cm⁻¹ | ν CH (alkyles) |
| 1620 | cm⁻¹ | ν CO (amide) |
| 1590 | cm⁻¹ | ν C=C (aromatiques) |
| 1250 | cm⁻¹ | ν OCH₃ |

| **Analyse spectroscopique de RMN du proton (DMSO d**_{**6**}**, δ, 300 MHz) :** | | | |
|---|---|---|---|
| 0,87 | PPm (triplet, 3H) | Hᵢ | Ji-h = 7,29 Hz |
| 1,26 | ppm (multiplet, 2H) | Hₕ | |
| 1,50 | ppm (multiplet, 2H) | H_{g} | |
| 1,81 | ppm (multiplet, 2H) | H_{c} | |
| 2,09 | ppm (triplet, 2H) | H_{f} | Jf-g = 7,39 Hz |
| 3,00 | ppm (triplet, 2H) | H_{b} | Jb-c = 7,68 Hz |
| 3,16 | ppm (multiplet, 2H) | H_{d} | |
| 3,93 | ppm (singulet, 3H) | Hₐ | |
| 7,16-7,35 | ppm (massif, 4H) | H_{2,3,6,8} | |
| 7,70 | ppm (doublet, 1H) | H₄ | Jortho = 7,83 Hz |
| 7,83 | ppm (doublet, 1H) | H₅ | Jortho = 8,94 Hz |
| 7,90 | ppm (signal, 1H) | Hₑ | |

### EXEMPLE 4 : N-PROPYL N'-[3-(7-METHOXYNAPHT-1-YL)PROPYL]UREE

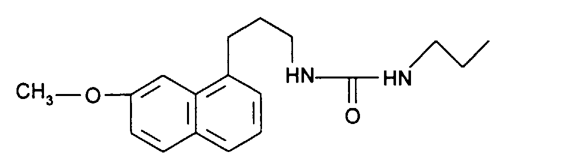

En partant de l'amine de la préparation 1 comme dans l'exemple 1 mais en la faisant réagir avec l'isocyanate de propyle au lieu du chlorure d'acétyle, on obtient le composé du titre. Point de fusion : 121°C

### EXEMPLES 5 A 19 :

En procédant comme dans l'exemple 1 mais en remplaçant le chlorure d'acétyle par le chlorure d'acyle approprié, on obtient les composés des exemples suivants :

### EXEMPLE 5 : N-[3-(7-METHOXY NAPTH-1-YL)PROPYL]PROPIONAMIDE

### EXEMPLE 6 : N-[3-(7-METHOXY NAPTH-1-YL)PROPYL]BUTANAMIDE

### EXEMPLE 7 : N-[3-(7-METHOXY NAPTH-1-YL)PROPYL]HEXANAMIDE

### EXEMPLE 8 : N-[3-(7-METHOXY NAPTH-1-YL)PROPYL]HEPTANAMIDE

### EXEMPLE 9 : N-[3-(7-METHOXY NAPTH-1-YL)PROPYL]ISOPROPYL CARBOXAMIDE

### EXEMPLE 10 : N-[3-(7-METHOXY NAPTH-1-YL)PROPYL]BROMOACETAMIDE

### EXEMPLE 11 : N-[3-(7-METHOXY NAPTH-1-YL)PROPYL]CYCLOBUTANE CARBOXAMIDE

### EXEMPLE 12 : N-[3-(7-METHOXY NAPTH-1-YL)PROPYL]CYCLOPENTANE CARBOXAMIDE

### EXEMPLE 13 : N-[3-(7-METHOXY NAPTH-1-YL)PROPYL]CYCLOHEXANE CARBOXAMIDE

### EXEMPLE 14 : N-[3-(7-METHOXY NAPTH-1-YL)PROPYL]CYCLOPROPYLb ACETAMIDE

### EXEMPLE 15 : N-[3-(7-METHOXY NAPTH-1-YL)PROPYL]CYCLOBUTYLACETAMIDE

### EXEMPLE 16 : N-[3-(7-METHOXY NAPTH-1-YL)PROPYL]VINYLCARBOXAMIDE

### EXEMPLE 17 : N-[3-(7-METHOXY NAPTH-1-YL)PROPYL]ALLYLCARBOXAMIDE

### EXEMPLE 18 : N-[3-(7-METHOXY NAPTH-1-YL)PROPYL]PROPARGYL CARBOXAMIDE

### EXEMPLE 19 : N-[3-(7-METHOXY NAPTH-1-YL)PROPYL]CROTYLCARBOXAMIDE

### EXEMPLES 20 A 24 :

En procédant comme dans l'exemple 4 mais en remplaçant l'isocyanate de propyle par l'isocyanate d'alkyle approprié, on obtient les composés suivants :

### EXEMPLE 20 : N-METHYL N'-[3-(7-METHOXY NAPTH-1-YL)PROPYL]UREE

### EXEMPLE 21 : N-ETHYL N'-[3-(7-METHOXY NAPTH-1-YL)PROPYL]UREE

### EXEMPLE 22 : N-BUTYL N'-[3-(7-METHOXY NAPTH-1-YL)PROPYL]UREE

### EXEMPLE 23 : N-PENTYL N'-[3-(7-METHOXY NAPTH-1-YL)PROPYL]UREE

### EXEMPLE 24 : N-CYCLOPROPYL N'-[3-(7-METHOXY NAPTH-1-YL)PROPYL]UREE

### EXEMPLES 25 A 27 :

Les composés des exemples 1, 2 et 3 sont mis à réagir avec le réactif de Lawesson afin d'obtenir respectivement les composés des exemples suivants :

### EXEMPLE 25 : N-[3-(7-METHOXY NAPTH-1-YL)PROPYL]THIOACETAMIDE

### EXEMPLE 26 : N-[3-(7-METHOXY NAPTH-1-YL)PROPYL]CYCLOPROPANE THIOCARBOXAMIDE

### EXEMPLE 27 : N-[3-(7-METHOXY NAPTH-1-YL)PROPYL]THIOPENTANAMIDE

### EXEMPLES 28 A 30 :

En procédant comme dans l'exemple 4 mais en remplaçant l'isocyanate de propyle par l'isothiocyanate d'alkyle approprié, on obtient les produits suivants :

### EXEMPLE 28 : N-METHYL N'-[3-(7-METHOXY NAPTH-1-YL)PROPYL]THIOUREE

### EXEMPLE 29 : N-ETHYL N'-[3-(7-METHOXY NAPTH-1-YL)PROPYL]THIOUREE

### EXEMPLE 30 : N-PROPYL N'-[3-(7-METHOXY NAPTH-1-YL)PROPYL]THIOUREE

### EXEMPLE 31 : N-METHYL N-[3-(7-METHOXY NAPTH-1-YL)PROPYL]ACETAMIDE

En procédant comme dans l'exemple 1 mais en utilisant au départ le chlorhydrate de N-méthyl N-[3-(7-méthoxy napht-1-yl)propyl]amine (préparation 16), on obtient le composé du titre.

### EXEMPLES 32 A 38 :

En procédant comme dans l'exemple 1 mais en utilisant au départ le chlorhydrate de la 3-(7-alkoxy napth-1-yl)propylamine appropriée obtenue aux préparations 17 à 23, on obtient respectivement les produits suivants :

### EXEMPLE 32 : N-[3-(7-ETHOXY NAPTH-1-YL)PROPYL]ACETAMIDE

### EXEMPLE 33 : N-[3-(7-PROPOXY NAPTH-1-YL)PROPYL]ACETAMIDE

### EXEMPLE 34 : N-[3-(7-BUTYLOXY NAPTH-1-YL)PROPYL]ACETAMIDE

### EXEMPLE 35 : N-[3-(7-PENTOXY NAPTH-1-YL)PROPYL]ACETAMIDE

### EXEMPLE 36 : N-[3-(7-HEXYLOXY NAPTH-1-YL)PROPYL]ACETAMIDE

### EXEMPLE 37 : N-[3-(7-CYCLOHEXYLOXY NAPTH-1-YL)PROPYL]ACETAMIDE

### EXEMPLE 38 : N-[3-(7-CYCLOPROPYLMETHOXY NAPTH-1-YL)PROPYL]ACETAMIDE

### EXEMPLES 39 A 44 :

En procédant comme dans l'exemple 1 mais en utilisant au départ le chorhydrate de la 3-(7-alkyl napht-1-yl)propylamine appropriée obtenue aux préparations 24 à 29, on obtient respectivement les produits suivants :

### EXEMPLE 39 : N-[3-(7-METHYL NAPTH-1-YL)PROPYL] ACETAMIDE

### EXEMPLE 40 : N-[3-(7-ETHYL NAPTH-1-YL)PROPYL] ACETAMIDE

### EXEMPLE 41 : N-[3-(7-PROPYL NAPTH-1-YL)PROPYL] ACETAMIDE

### EXEMPLE 42 : N-[3-(7-BUTYL NAPTH-1-YL)PROPYL] ACETAMIDE

### EXEMPLE 43 : N-[3-(7-PENTYL NAPTH-1-YL)PROPYL] ACETAMIDE

### EXEMPLE 44 : N-[3-(7-HEXYL NAPTH-1-YL)PROPYL] ACETAMIDE

### EXEMPLES 45 A 56 :

En procédant comme dans l'exemple 1 mais en utilisant au départ les composés des préparations 2 à 13, on obtient respectivement les produits suivants :

### EXEMPLES 45 : N-[3-(7-ETHYL-1,2,3,4-TETRAHYDRONAPHT-1-YL)PROPYL] ACETAMIDE

### EXEMPLES 46 : N-[3-(5-ETHYL-BENZOTHIOPHEN-3-YL)PROPYL]ACETAMIDE

### EXEMPLES 47 : N-[3-(7-METHYL-1,2,3,4-TETRAHYDRONAPHT-1-YL)PROPYL] ACETAMIDE

### EXEMPLES 48 : N-[3-(7-PROPYL-1,2,3,4-TETRAHYDRONAPHT-1-YL)PROPYL] ACETAMIDE

### EXEMPLES 49 : N-[3-(7-BUTYL-1,2,3,4-TETRAHYDRONAPHT-1-YL)PROPYL] ACETAMIDE

### EXEMPLE 50 : N-[3-(5-METHYL-BENZOFURAN-3-YL)PROPYL]ACETAMIDE

### EXEMPLE 51 : N-[3-(5-ETHYL-BENZOFURAN-3-YL)PROPYL]ACETAMIDE

### EXEMPLE 52 N-[3-(7-METHOXY-1,2,3,4-TETRAHYDRONAPHT-1-YL)PROPYL] ACETAMIDE

### EXEMPLE 53 : N-[3-(5-METHOXY-BENZOFURAN-3-YL)PROPYL]ACETAMIDE

### EXEMPLES 54 : N-[3-(NAPHT-1-YL)PROPYL]ACETAMIDE

### EXEMPLE 55 : N-[3-(5-METHOXY-BENZOTHIOPHEN-3-YL)PROPYL] ACETAMIDE

### EXEMPLE 56 : N-[3-(5-METHOXY-INDOL-3-YL)PROPYL]ACETAMIDE

### EXEMPLE 57 : N-[3-(5-METHYL-BENZOTHIOPHEN-3-YL)PROPYL] CYCLOPROPANECARBOXAMIDE

En procédant comme dans l'exemple 2 mais en utilisant au départ le composé obtenu à la préparation 14, on obtient le produit du titre.

### EXEMPLES 58 ET 59 :

En procédant comme dans l'exemple 4 mais en utilisant au départ les composés des préparations 2 et 3, on obtient respectivement les produits suivants :

### EXEMPLES 58 : N-PROPYL N'-[3-(7-ETHYL-1,2,3,44ETRAHYDRONAPHT-1-YL) PROPYL]UREE

### EXEMPLES 59 : N-PROPYL N'-[3-(5-ETHYL-BENZOTHIOPHEN-3-YL)PROPYL]UREE

### EXEMPLES 60 A 63 :

En procédant comme dans les exemples 1 à 4 mais en utilisant au départ le composé obtenu à la préparation 15, on obtient respectivement les produits suivants :

### EXEMPLE 60 N-[4-(7-METHOXY NAPHT-1-YL)BUTYL]ACETAMIDE

### EXEMPLE 61 : N-[4-(7-METHOXY NAPHT-1-YL)BUTYL]CYCLOPROPANE CARBOXAMIDE

### EXEMPLE 62 : N-[4-(7-METHOXY NAPHT-1-YL)BUTYL]PENTANAMIDE

### EXEMPLE 63 : N-PROPYL N'-[4-(7-METHOXY NAPHT-1-YL)BUTYL]UREE

### EXEMPLE 64 : N-[6-(7-METHOXY NAPHT-1-YL)HEXYL]ACETAMIDE

En procédant comme dans l'exemple 1 mais en utilisant au départ le composé obtenu à la préparation 30, on obtient le produit du titre.

### EXEMPLE 65 : N-[3-(7-METHOXY 3-BENZOYL NAPHT-1-YL)PROPYL]ACETAMIDE

En faisant réagir le N-[3-(7-méthoxy napht-1-yl)propyl]acétamide obtenu à l'exemple 1 avec du chlorure de benzoyle, on obtient le composé du titre.

### EXEMPLE 66 : N-[3-(7-METHOXY 3-BENZYL NAPHT-1-YL)PROPYL]ACETAMIDE

Par réduction du composé de l'exemple 65 par le mercure et le zinc, on obtient le composé du titre.

### EXEMPLE 67 : N-[3-(7-METHOXY 3-ACETYL NAPHT-1-YL)PROPYL]ACETAMIDE

En faisant réagir le N-[3-(7-méthoxy napht-1-yl)propyl]acétamide obtenu à l'exemple 1 avec du chlorure d'acétyle, on obtient le composé du titre.

### EXEMPLE 68 : N-[3-(7-METHOXY 3-ETHYL NAPHT-1-YL)PROPYL]ACETAMIDE

Par réduction du composé de l'exemple 67 par le mercure et le zinc, on obtient le composé du titre.

### ETUDE PHARMACOLOGIQUE

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée.

La DL 50 des produits testés est supérieure à 1000 mg.kg⁻¹ pour la plupart des composés étudiés ce qui indique la faible toxicité des composés de l'invention.

### EXEMPLE B : ETUDE DE LIAISON AUX RECEPTEURS DE LA MELATONINE

### B1) ETUDE SUR DES CELLULES DE LA PARS TUBERALIS DE MOUTON

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology vol. (1), pp 14 (1989)).

### PROTOCOLE

1) Les membranes de pars tuberalis de mouton sont préparées et utilisées comme tissu cible dans des expériences de saturation pour déterminer les capacités et affinités de liaison pour la 2-[¹²⁵I] - iodomélatonine.

2) Les membranes de pars tuberalis de mouton sont utilisées comme tissu cible, avec les différents composés à tester, dans des expériences de liaison compétitive par rapport à la 2-[¹²⁵I] - iodomélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

Il apparaît que les composés de l'invention possèdent une puissante affinité pour les récepteurs de la mélatonine supérieure à la mélatonine elle-même.

### B2) ETUDE SUR DES MEMBRANES DE CELLULES DU CERVEAU DE POULET (GALLUS DOMESTICUS)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 128, pages 475-482, 1991). La [¹²⁵I] mélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2-[¹²⁵I] -iodomélatonine
- mélatonine
- produits courants
- molécules originales

En screening primaire, les molécules sont testées à 2 concentrations (10⁻⁷ et 10⁻⁵ M). Chaque résultat est la moyenne de 3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention, qui correspondent aux valeurs de l'affinité montrent que la liaison des composés testés est très puissante.

### EXEMPLE C : TEST DES QUATRE PLAQUES

Les produits de l'invention sont administrés par voie oesophagienne à des lots de dix souris. Un lot reçoit du sirop de gomme. 30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE D : COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE EXPERIMENTAL

Des rats mâles Long Evans âgés de un mois sont soumis dès leur arrivée au laboratoire à un cycle lumineux de 12h de lumière par 24h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

Il apparaît clairement que les composés de l'invention permettent d'agir de façon puissante sur le rythme circadien via le système mélatoninergique.

### EXEMPLE E : ACTIVITE ANTIARYTHMIQUE

### PROTOCOLE :

### (Ref: LAWSON J.W. et al. J. Pharmacol. Expert. Therap. 1968, 160, pp 22-31)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F : COMPOSITION PHARMACEUTIQUE : COMPRIMES

| 1000 comprimés dosés à 5 mg de N-[3-(7-méthoxy napht-1-yl)propyl]acétamide | |
|---|---|
| N-[3-(7-méthoxy napht-1-yl)propyl]acétamide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 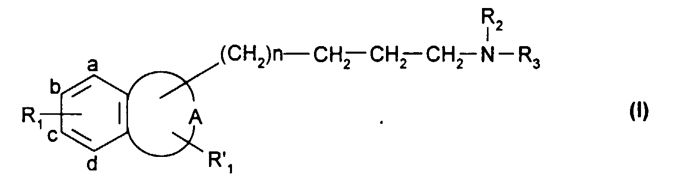 dans laquelle :
- R₁ représente un hydrogène, un hydroxy, un radical R₆ ou un groupement -O-R₆, R₆ étant choisi parmi alkyle, alkyle substitué, cycloalkyle, cycloalkyle substitué, cycloalkylalkyle, cycloalkylalkyle substitué, alcényle, alcynyle, cycloalcényle, phényle, phényle substitué, phénylalkyle, phénylalkyle substitué, dicycloalkylalkyle, dicycloalkylalkyle substitué, diphénylalkyle et diphénylalkyle substitué,
- R'₁ représente un hydrogène, un halogène, ou un groupement choisi parmi :
- -R'₆,
- -O-R'₆,
- -OH,
- -CO-R₇,
- -CH₂-R₇,
- -O-CO-R₇,
R'₆ étant choisi parmi les mêmes valeurs que R₆, qui est tel que défini précédemment, les radicaux R₆ et R'₆ étant identiques ou différents,
R₇ représentant un radical choisi parmi (C₁-C₅)alkyle, (C₁-C₅)alkyle substitué, cycloalkyle, cycloalkyle substitué, cycloalkyl(C₁-C₅)alkyle, cycloalkyl(C₁-C₅)alkyle substitué, phényle, phényle substitué, phényl(C₁-C₅)alkyle. et phényl(C₁-C₅)alkyle substitué,
- A forme avec le noyau benzénique auquel il est lié un groupement cyclique choisi parmi tétrahydronaphtalène, dihydronaphtalène, naphtalène, benzothiophène, 2,3-dihydrobenzothiophène, benzofurane, 2,3-dihydrobenzofurane, indole et indoline,
- n représente zéro, 1, 2 ou 3,
- R₂ représente un hydrogène ou un alkyle,
- R₃ représente :
• un groupement de formule (R₃₁) : 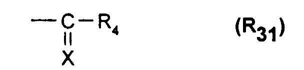 avec X représentant un soufre ou un oxygène et R₄ représentant un hydrogène ou un radical choisi parmi alkyle, alkyle substitué, alcényle, alcynyle, cycloalkyle, cycloalkyle substitué, cycloalkylalkyle et cycloalkylalkyle substitué,
• ou un groupement de formule (R₃₂) : 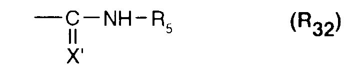
avec X' représentant un soufre ou un oxygène et R₅ représentant un hydrogène ou un radical non substitué ou substitué choisi parmi alkyle, cycloalkyle, et cycloalkylalkyle,
le composé de formule (I) ne pouvant pas représenter le N-[3-(4-hydroxy-napht-1-yl) propyl]acétamide ou le N-[3-(4-benzyloxy-napht-1-yl)propyl]acétamide,
étant entendu que :
- lorsque A forme avec le noyau benzénique auquel il est lié un groupement benzothiophène, n est égal à zéro, R'₁ et R₂ représentent simultanément un atome d'hydrogène, R₁ représente un atome d'hydrogène, un groupement hydroxy un groupement alkyle ou un groupement alkoxy et X représente un atome d'oxygène, alors R₄ ne peut représenter un atome d'hydrogène ou un groupement alkyle,
- lorsque A forme avec le noyau benzénique auquel il est lié un groupement benzothiophène, R₄ est un radical alkyle ou vinyle, R₁ est un hydrogène ou un méthyle et R'₁ est un hydrogène, alors n est différent de zéro,
- lorsque A forme avec le noyau benzènique auquel il est lié un groupement indole, n est égal à zéro, R₂ représente un hydrogène, R'₁ représente un hydrogène ou radical phényle, méthyle ou benzyle, substitué sur l'azote du groupement indole formé par A, et R₁ représente un hydrogène ou un groupement méthyle alors R₄ ne peut pas être un radical méthyle ou éthyle,
- le terme "alkyle" désigne un groupement linéaire ou ramifié contenant de 1 à 6 atomes de carbone,
- les termes "alcényle" et "alcynyle" désignent des groupements linéaires ou ramifiés contenant de 2 à 6 atomes,
- le terme "cycloalkyle" désigne un groupement de 3 à 8 atomes de carbone,
- le terme "cycloalcényle" désigne un groupement insaturé de 5 à 8 atomes de carbone,
- le terme "substitué" associé au radical "alkyle" signifie que ce radical est substitué par un ou plusieurs substituants choisis parmi halogène, hydroxy et alkoxy,
- le terme "substitué" associé aux radicaux cycloalkyle, cycloalkylalkyle, dicycloalkylalkyle signifie que ces radicaux sont substitués sur la partie cycloalkyle par un ou plusieurs groupements choisis parmi halogène, alkyle, hydroxy, alkoxy et oxo,
- et le terme "substitué" associé aux radicaux phényle, phénylalkyle, phényl(C₁-C₅)alkyle, diphénylalkyle signifie que ces radicaux sont substitués sur la partie phényle par un ou plusieurs groupements choisis parmi halogène, hydroxy, alkyle, alkyle substitué par un ou plusieurs halogènes et alkoxy,
leurs énantiomères et diastéréoisomères,
et leurs sels d'addition avec une base pharmaceutiquement acceptable.

2. Composés selon la revendication 1 de formule (1) à (12), cas particuliers des composés de formule (I) selon la revendication 1 : 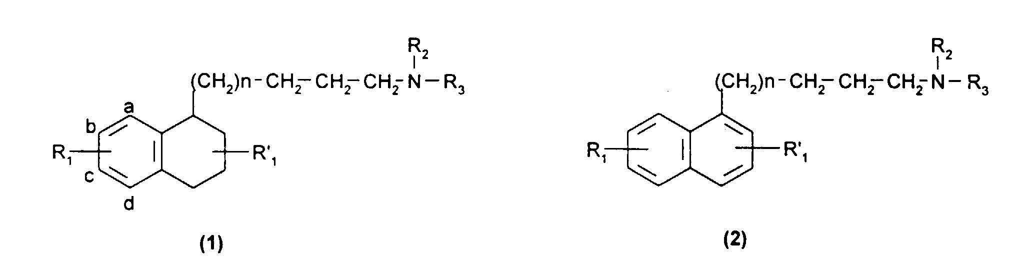 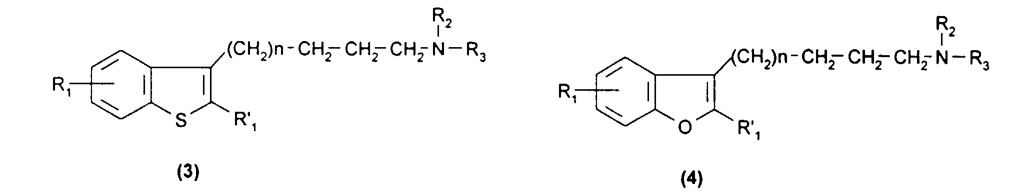 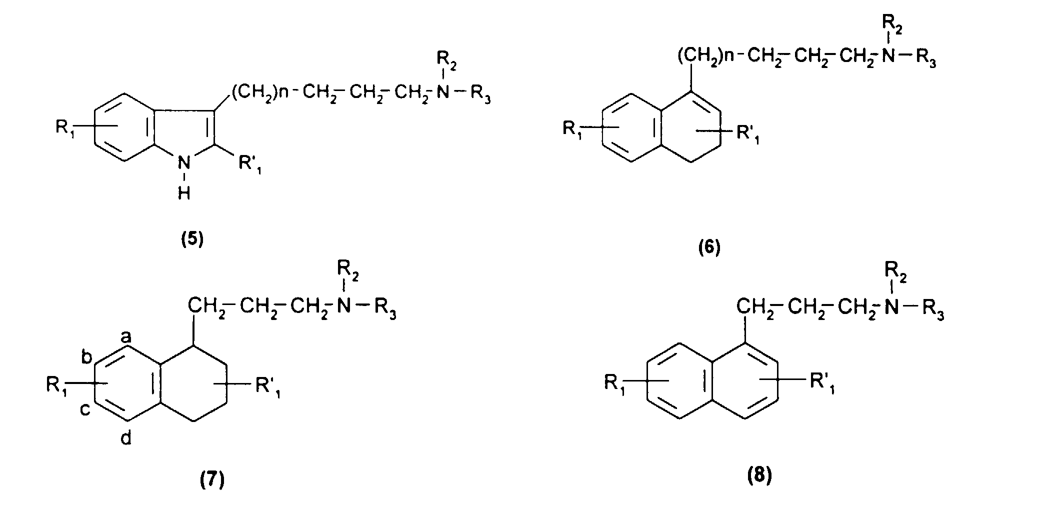 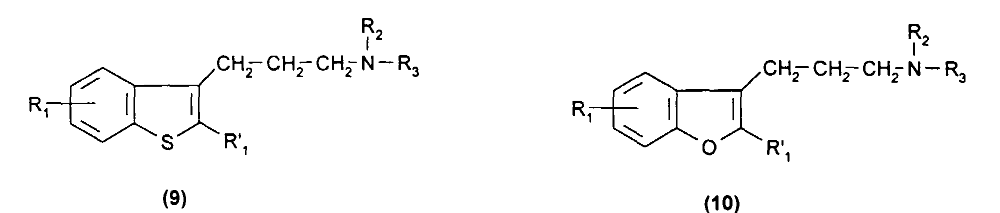 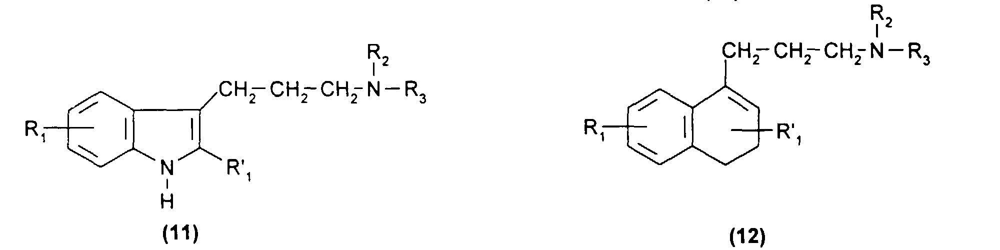 dans lesquelles R₁, R'₁, R₂, R₃ et n sont tels que défini dans la revendication 1.

3. Composés de formule (I) selon la revendication 1 dans laquelle R'₁ est un hydrogène.

4. Composé de formule (I) selon la revendication 1 qui est le N-[3-(7-méthoxynapht-1-yl)propyl]acétamide,

5. Composé de formule (I) selon la revendication 1 qui est le N-[3-(7-méthoxynapht-1-yl)propyl]pentanamide,

6. Composé de formule (I) selon la revendication 1 qui est le N-[3-(7-méthoxynapht-1-yl)propyl]cyclopropylcarboxamide,

7. Composé de formule (I) selon la revendication 1 qui est le N-propyl N'-[3-(7-méthoxynapht-1-yl)propyl]urée,

8. Procédé de préparation des composés de formule (I) selon la revendication 1 caractérisé en ce que on fait réagir un composé de formule (II) : 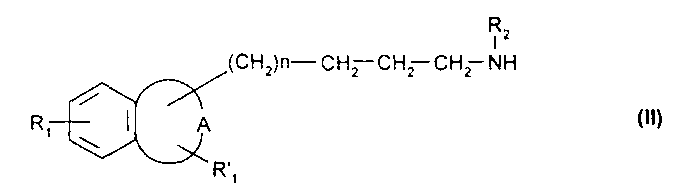 dans laquelle R₁, R'₁, R₂, n et A sont tels que définis dans la revendication 1,
- soit avec un composé de formule (IIIa) ou (IIIb) : 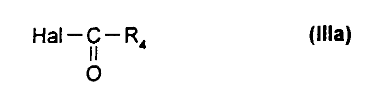 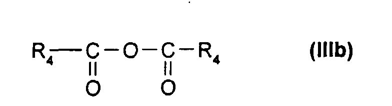 l'anhydride de formule (IIIb) étant mixte ou symétrique,
dans laquelle R₄ est tel que défini dans la revendication 1 et Hal représente un halogène, afin d'obtenir les composés de formule (I/a) : 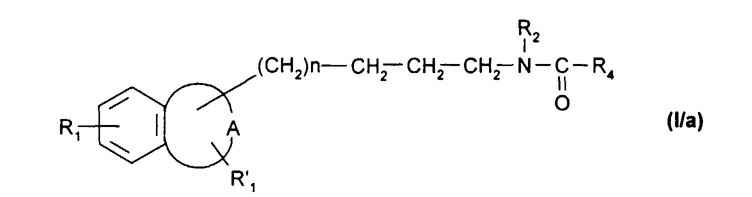 dans laquelle R₁, R'₁, R₂, R₄, A et n sont tels que définis précédemment,
composés de formule (I/a) qui sont soumis à un agent de thionation, par exemple le réactif de Lawesson, pour obtenir les composés de formule (I/a') : 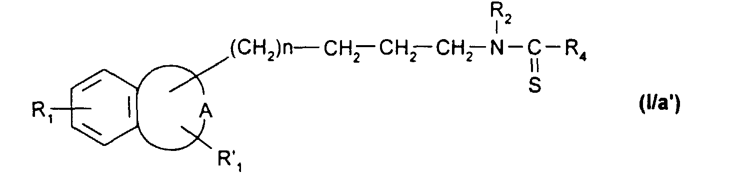 dans laquelle R₁, R'₁, R₂, R₄, A, et n, sont tels que définis précédemment,
- soit avec un composé de formule (IV) :
X'=C=N-R₅ **(IV)**
dans laquelle X' et R₅ sont tels que définis dans la revendication 1
afin d'obtenir les composés de formule (I/b) : 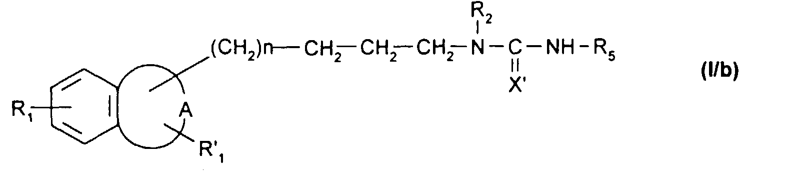 dans laquelle R₁, R_{1'}, R₂, R₅, A, n, et X' sont tels que définis précédemment, les composés de formule (I/a), (I/a') et (I/b) formant l'ensemble des composés de formule (I) selon la revendication 1 pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

9. Compositions pharmaceutiques contenant les produits de formule (I) selon la revendication 1 ou un de leurs sels d'addition avec une base pharmaceutiquement acceptable en combinaison avec un ou plusieurs excipients pharmaceutiquement acceptables.

10. Compositions selon la revendication 9 utiles pour le traitement des troubles du système mélatoninergique.

## Patentansprüche

1. Verbindungen der Formel (I): 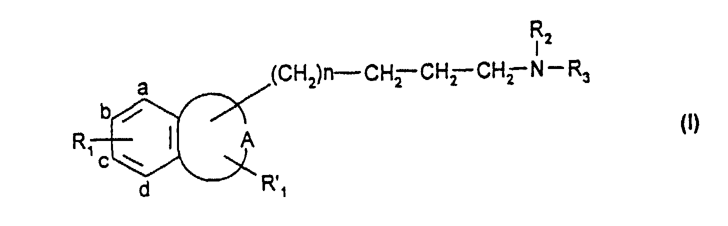 in der:
- R₁ ein Wasserstoffatom, eine Hydroxygruppe, eine Gruppe R₆ oder eine Gruppe -O-R₆ bedeutet, worin R₆ aus Alkyl, substituiertem Alkyl, Cycloalkyl, substituiertem Cycloalkyl, Cycloalkylalkyl, substituiertem Cycloalkylalkyl, Alkenyl, Alkinyl, Cycloalkenyl, Phenyl, substituiertem Phenyl, Phenylalkyl, substituiertem Phenylalkyl, Dicycloalkylalkyl, substituiertem Dicycloalkylalkyl, Diphenylalkyl und substituiertem Diphenylalkyl ausgewählt ist,
- R'₁ ein Wasserstoffatom, ein Halogenatom oder eine Gruppe ausgewählt aus:
- -R'₆,
- -O-R'₆,
- -OH,
- -CO-R₇,
- -CH₂-R₇,
- -O-CO-R₇,
worin R'₆ aus den gleichen Gruppen ausgewählt ist wie sie oben für R₆ definiert worden sind, wobei die Gruppen R₆ und R'₆ gleichartig oder verschieden sind, R₇ eine Gruppe ausgewählt aus (C₁-C₅)-Alkyl, substituiertem (C₁-C₅)-Alkyl, Cycloalkyl, substituiertem Cycloalkyl, Cycloalkyl-(C₁-C₅)-alkyl, substituiertem Cycloalkyl-(C₁-C₅)-alkyl, Phenyl, substituiertem Phenyl, Phenyl-(C₁-C₅)-alkyl und substituiertem Phenyl-(C₁-C₅)-alkyl bedeutet, darstellt,
- A mit dem Benzolkern, an den sie gebunden ist, eine cyclische Gruppe ausgewählt aus Tetrahydronaphthalin, Dihydronaphthalin, Naphthalin, Benzothiophen, 2,3-Dihydrobenzothiophen, Benzofuran, 2,3-Dihydrobenzofuran, Indol und Indolin darstellt,
- n Null, 1, 2 oder 3 bedeutet,
- R₂ ein Wasserstoffatom oder eine Alkylgruppe darstellt,
- R₃:
• eine Gruppe der Formel (R₃₁): 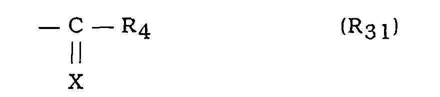 worin X Sauerstoff oder Schwefel und R₄ ein Wasserstoffatom oder eine Gruppe ausgewählt aus Alkyl, substituiertem Alkyl, Alkenyl, Alkinyl, Cycloalkyl, substituiertem Cycloalkyl, Cycloalkylalkyl und substituiertem Cycloalkylalkyl darstellen,
• oder eine Gruppe der Formel (R₃₂): 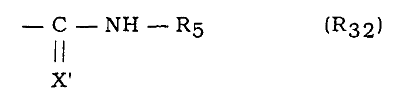 bedeutet, worin X' Schwefel oder Sauerstoff und R₅ ein Wasserstoffatom oder eine nicht substituierte oder substituierte Gruppe ausgewählt aus Alkyl, Cycloalkyl und Cycloalkylalkyl bedeuten,
wobei die Verbindung der Formel (I) nicht N-[3-(4-Hydroxy-naphth-1-yl)-propyl]-acetamid oder N-[3-(4-Benzyloxy-naphth-1-yl)-propyl]-acetamid bedeutet,
mit der Maßgabe, daß:
- wenn A mit dem Benzolkern, an den sie gebunden ist, eine Benzothiophengruppe darstellt, n Null ist, R'₁ und R₂ gleichzeitig ein Wasserstoffatom darstellen, R₁ ein Wasserstoffatom, eine Hydroxygruppe, eine Alkylgruppe oder eine Alkoxygruppe darstellt und X ein Sauerstoffatom bedeutet, während R₄ nicht ein Wasserstoffatom oder eine Alkylgruppe darstellt,
- wenn A mit dem Benzolkern, an den sie gebunden ist, eine Benzothiophengruppe darstellt, R₄ eine Alkylgruppe oder Vinylgruppe, R₁ ein Wasserstoffatom oder eine Methylgruppe und R'₁ ein Wasserstoffatom bedeuten, während n von Null verschieden ist,
- wennA zusammen mit dem Benzolkern, an den sie gebunden ist, eine Indolgruppe darstellt, n Null ist, R₂ ein Wasserstoffatom, R'₁ ein Wasserstoffatom oder eine Phenylgruppe, Methylgruppe oder Benzylgruppe darstellen, die am Stickstoffatom der durch A gebildeten Indolgruppe substituiert ist, und R₁ ein Wasserstoffatom oder eine Methylgruppe darstellt, während R₄ nicht eine Methyl- oder Ethylgruppe bedeutet,
- der Begriff "Alkyl" für eine geradkettige oder verzweigte Gruppe steht, die 1 bis 6 Kohlenstoffatome enthält,
- die Begriffe "Alkenyl" und "Alkinyl" für geradkettige oder verzweigte Gruppen stehen, die 2 bis 6 Kohlenstoffatome enthalten,
- der Begriff "Cycloalkyl" für eine Gruppe mit 3 bis 8 Kohlenstoffatomen steht,
- der Begriff "Cycloalkenyl" für eine ungesättigte Gruppe mit 5 bis 8 Kohlenstoffatomen steht,
- der Begriff "substituiert" unter Bezugnahme auf eine "Alkylgruppe" bedeutet, daß diese Gruppe durch einen oder mehrere Substituenten ausgewählt aus Halogen, Hydroxy und Alkoxy substituiert ist,
- der Begriff "substituiert" unter Bezugnahme auf Cycloalkyl-, Cycloalkylalkyl- und Dicycloalkylalkylgruppen bedeutet, daß diese Gruppen am Cycloalkylrest durch eine oder mehrere Gruppen ausgewählt aus Halogen, Alkyl, Hydroxy, Alkoxy und Oxo substituiert sind,
- und der Begriff"substituiert" unter Bezugnahme aufPhenyl-, Phenylalkyl-, Phenyl-(C₁-C₅)-alkyl- und Diphenylalkylgruppen bedeutet, daß diese Gruppen am Phenylrest durch eine oder mehrere Gruppen ausgewählt aus Halogen, Hydroxy, Alkyl und durch ein oder mehrere Halogenatome substituiertem Alkyl und Alkoxygruppen substituiert sind,
deren Enantiomere und Diastereoisomere,
und deren Additionssalze mit einer pharmazeutisch annehmbaren Base.

2. Verbindungen nach Anspruch 1 der Formeln (1) bis (12), welche Sonderfälle der Verbindungen der Formel (I) nach Anspruch 1 darstellen: 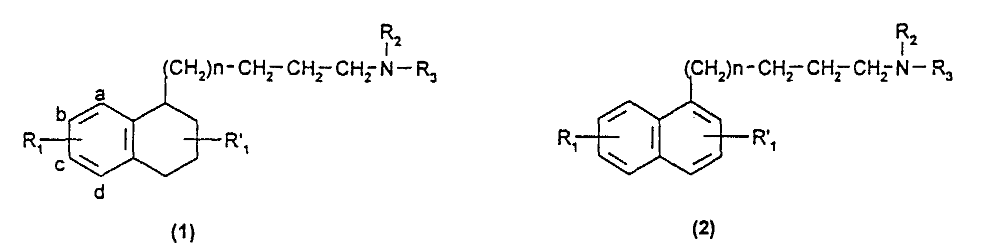 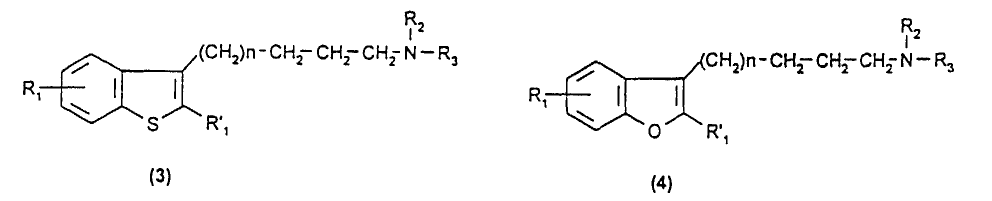 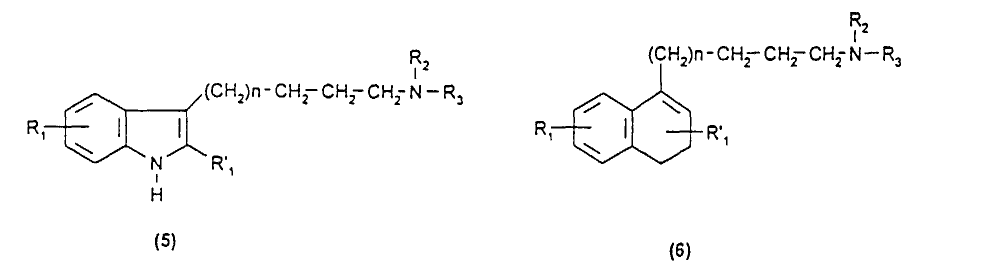 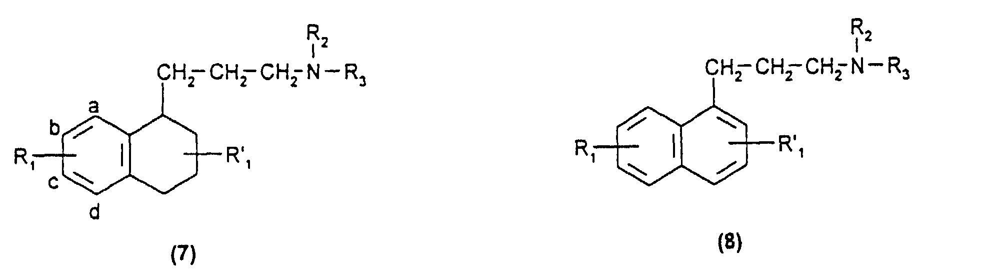 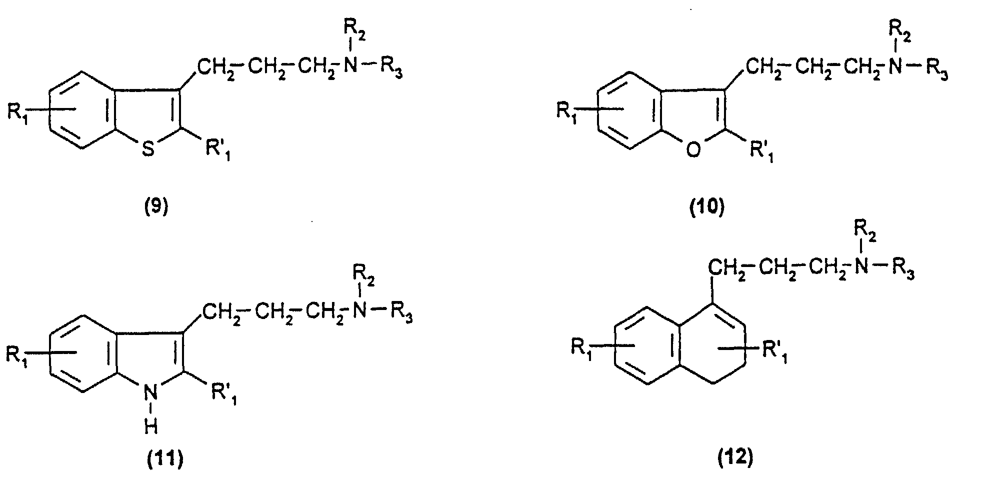 worin R₁, R'₁, R₂, R₃ und n die in Anspruch 1 angegebenen Bedeutungen besitzen.

3. Verbindungen der Formel (I) nach Anspruch 1, worin R'₁ ein Wasserstoffatom darstellt.

4. Verbindung der Formel (I) nach Anspruch 1, nämlich N-[3-(7-Methoxynaphth-1-yl)-propyl]-acetamid.

5. Verbindung der Formel (I) nach Anspruch 1, nämlich N-[3-(7-Methoxynaphth-1-yl)-propyl]-pentanamid.

6. Verbindung der Formel (I) nach Anspruch 1, nämlich N-[3-(7-Methoxynaphth-1-yl)-propyl]-cyclopropylcarboxamid.

7. Verbindung der Formel (I) nach Anspruch 1, nämlich N-Propyl-N'-[3-(7-methoxynaphth-1-yl)-propyl]-harnstoff.

8. Verfahren zur Herstellung der Verbindungen der Formel (I) nach Anspruch 1,
dadurch gekennzeichnet, daß man eine Verbindung der Formel (II): 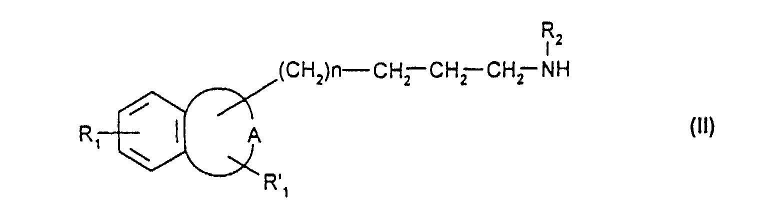 in der R₁, R'₁, R₂, n und A die in Anspruch 1 angegebenen Bedeutungen besitzen,
- entweder mit einer Verbindung der Formel (IIIa) oder (IIIb): 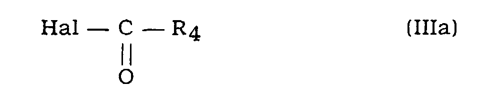 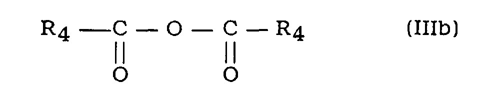 wobei das Anhydrid der Formel (IIIb) gemischt oder symmetrisch ist,
worin R₄ die in Anspruch 1 angegebenen Bedeutungen besitzt und Hal für ein Halogenatom steht,
umsetzt zur Bildung der Verbindungen der Formel (I/a): 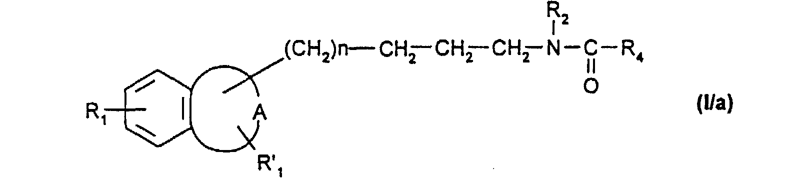 in der R₁, R'₁, R₂, R₄, A und n die oben angegebenen Bedeutungen besitzen, welche Verbindungen der Formel (I/a) der Einwirkung eines Thionierungsmittels, beispielsweise des Lawesson-Reagens, unterworfen werden zur Bildung der Verbindungen der Formel (I/a'): 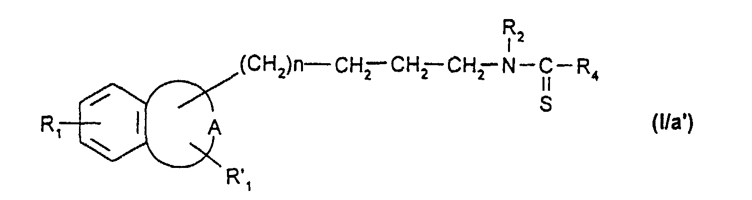 in der R₁, R'₁, R₂, R₄, A und n die oben angegebenen Bedeutungen besitzen,
- oder mit einer Verbindung der Formel (IV):
X' = C = N - R₅ (IV)
in der X' und R₅ die in Anspruch 1 angegebenen Bedeutungen besitzen, umgesetzt wird zur Bildung der Verbindungen der Formel (I/b): 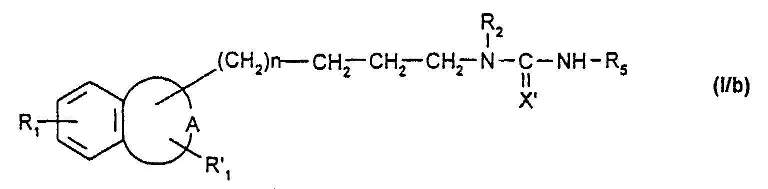 in der R₁, R'₁, R₂, R₅, A, n und X' die oben angegebenen Bedeutungen besitzen, welche Verbindungen der Formeln (I/a), (I/a') und (I/b), welche die Gesamtheit der Verbindungen der Formel (I) nach Anspruch 1 bilden, gewünschtenfalls
- mit Hilfe eines oder mehrerer Reinigungsverfahren ausgewählt aus Kristallisation, Chromatographie über Kieselgel, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können,
- oder mit einer pharmazeutisch annehmbaren Base in ihre Salze überführt werden können.

9. Pharmazeutische Zubereitungen enthaltend die Verbindungen der Formel (I) nach Anspruch 1 oder eines ihrer Additionssalze mit einer pharmazeutisch annehmbaren Base in Kombination mit einem oder mehreren pharmazeutisch annehmbaren Trägermaterialien.

10. Zubereitungen nach Anspruch 9 zur Behandlung von Störungen des melatoninergischen Systems.

## Claims

1. Compounds of formula (I): 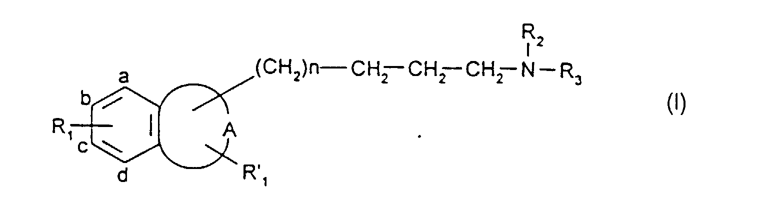 wherein:
- R¹ represents a hydrogen atom, hydroxy, an R₆ radical or an -O-R₆ group, with R₆ being selected from alkyl, substituted alkyl, cycloalkyl, substituted cycloalkyl, cycloalkylalkyl, substituted cycloalkylalkyl, alkenyl, alkynyl, cycloalkenyl, phenyl, substituted phenyl, phenylalkyl, substituted phenylalkyl, dicycloalkylalkyl, substituted dicycloalkylalkyl, diphenylalkyl and substituted diphenylalkyl,
- R'₁ represents a hydrogen atom, a halogen atom, or a group selected from:
- -R'₆,
- -O-R'₆,
- -OH,
- -CO-R₇,
- -CH₂-R₇,
- -O-CO-R₇,
R'₆ being selected from the same meanings as R₆, which is as defined hereinbefore, the R₆ and R'₆ radicals being identical or different,
R₇ representing a radical selected from (C₁-C₅)alkyl, substituted (C₁-C₅)alkyl, cycloalkyl, substituted cycloalkyl, cycloalkyl-(C₁-C₅)alkyl, substituted cycloalkyl-(C₁-C₅)alkyl, phenyl, substituted phenyl, phenyl-(C₁-C₅)alkyl and substituted phenyl-(C₁-C₅)alkyl,
- A forms with the benzene ring to which it is bonded a cyclic group selected from tetrahydronaphthalene, dihydronaphthalene, naphthalene, benzothiophene, 2,3-dihydrobenzothiophene, benzofuran, 2,3-dihydrobenzofuran, indole and indoline,
- n is zero, 1, 2 or 3,
- R₂ represents a hydrogen atom or an alkyl,
- R₃ represents:
• a group of formula (R₃₁): 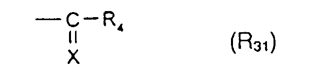 with X representing a sulphur or oxygen atom and R₄ representing a hydrogen atom or a radical selected from alkyl, substituted alkyl, alkenyl, alkynyl,
cycloalkyl, substituted cycloalkyl, cycloalkylalkyl and substituted cycloalkylalkyl,
• or a group of formula (R₃₂): 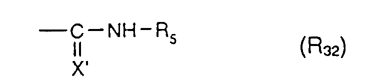 with X' representing a sulphur or oxygen atom and R₅ representing a hydrogen atom or an unsubstituted or substituted radical selected from alkyl, cycloalkyl and cycloalkylalkyl,
it not being possible for the compound of formula (I) to represent N-[3-(4-hydroxy-naphth-1-yl)propyl]acetamide or N-[3-(4-benzyloxy-naphth-1-yl)propyl]acetamide,
with the proviso that:
- when A forms with the benzene ring to which it is bonded a benzothiophene group, n is zero, R'₁ and R₂ simultaneously represent a hydrogen atom, R₁ represents a hydrogen atom, a hydroxy group, an alkyl group or an alkoxy group and X represents an oxygen atom, then R₄ cannot represent a hydrogen atom or an alkyl group,
- when A forms with the benzene ring to which it is bonded a benzothiophene group, R₄ is an alkyl or vinyl radical, R₁ is a hydrogen atom or methyl and R'₁ is a hydrogen atom, then n is other than zero,
- when A forms with the benzene ring to which it is bonded an indole group, n is zero, R₂ represents a hydrogen atom, R'₁ represents a hydrogen atom or a phenyl, methyl or benzyl radical substituted on the nitrogen of the indole group formed by A, and R₁ represents a hydrogen atom or a methyl group, then R₄ cannot be a methyl or ethyl radical,
it being understood that:
- the term "alkyl" denotes a linear or branched group containing from 1 to 6 carbon atoms,
- the terms "alkenyl" and "alkynyl" denote linear or branched groups containing from 2 to 6 carbon atoms,
- the term "cycloalkyl" denotes a group having from 3 to 8 carbon atoms,
- the term "cycloalkenyl" denotes an unsaturated group having from 5 to 8 carbon atoms,
- the term "substituted" applied to the radical "alkyl" means that that radical is substituted by one or more substituents selected from halogen, hydroxy and alkoxy,
- the term "substituted" applied to the cycloalkyl, cycloalkylalkyl and dicycloalkylalkyl radicals means that those radicals are substituted on the cycloalkyl moiety by one or more groups selected from halogen, alkyl, hydroxy, alkoxy and oxo, and
- the term "substituted" applied to the phenyl, phenylalkyl, phenyl-(C₁-C₅)alkyl and diphenylalkyl radicals means that those radicals are substituted on the phenyl moiety by one or more groups selected from halogen, hydroxy, alkyl, alkyl substituted by one or more halogens, and alkoxy,
their enantiomers and diastereoisomers,
and addition salts thereof with a pharmaceutically acceptable base.

2. Compounds according to claim 1 of formulae (1) to (12), particular cases of the compounds of formula (I) according to claim 1: 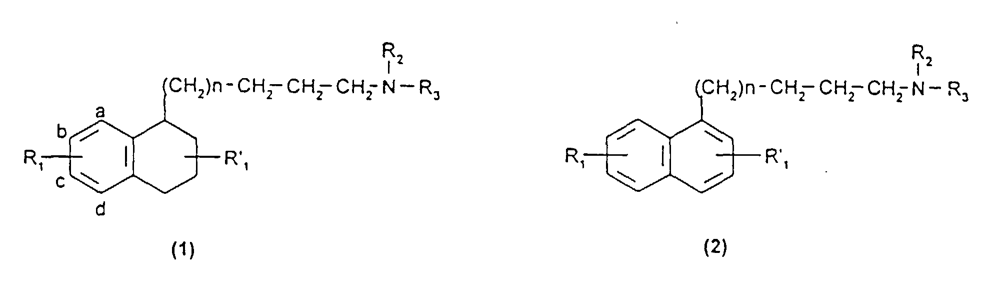 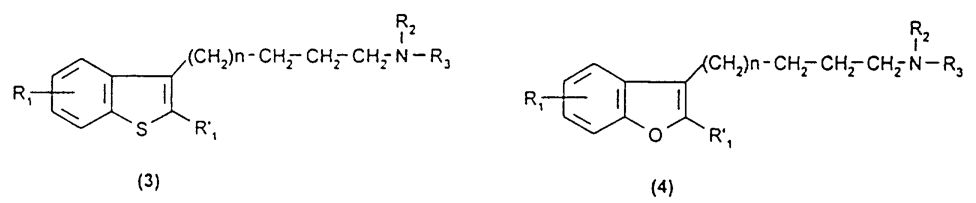 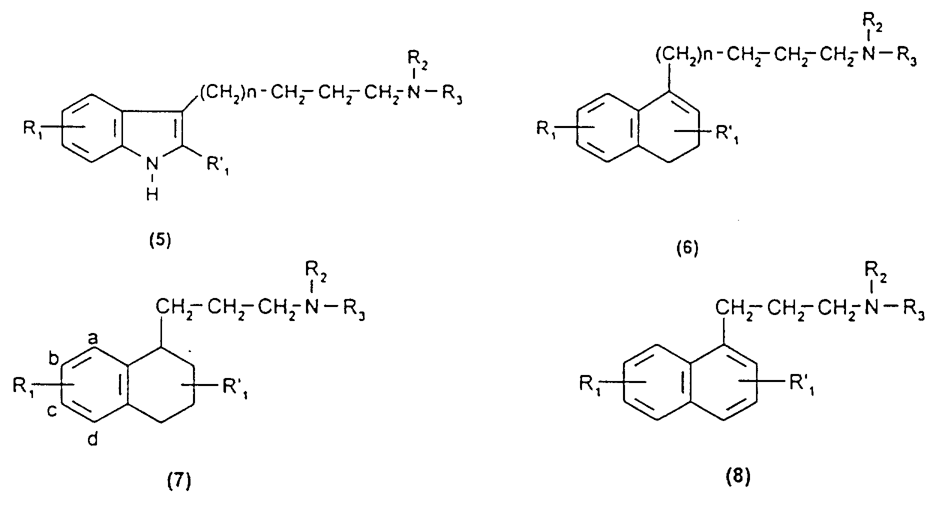 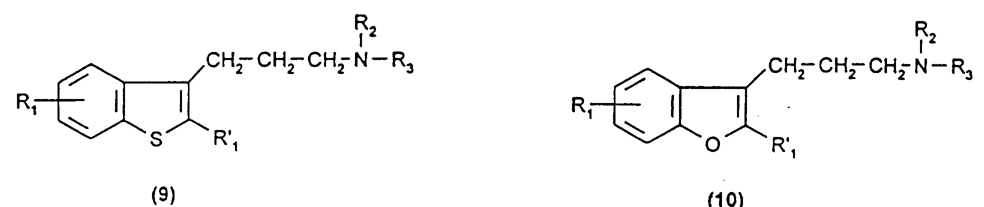 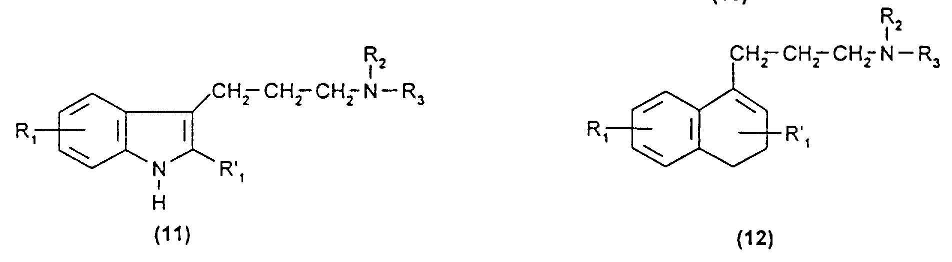 wherein R₁, R'₁, R₂, R₃ and n are as defined in claim 1.

3. Compounds of formula (I) according to claim 1 wherein R'₁ is a hydrogen atom.

4. Compound of formula (I) according to claim 1 which is N-[3-(7-methoxynaphth-1-yl)propyl]acetamide.

5. Compound of formula (I) according to claim 1 which is N-[3-(7-methoxynaphth-1-yl)propyl]pentanamide.

6. Compound of formula (I) according to claim 1 which is N-[3-(7-methoxynaphth-1-yl)propyl]cyclopropylcarboxamide.

7. Compound of formula (I) according to claim 1 which is N-propyl-N'-[3-(7-methoxynaphth-1-yl)propyl]urea.

8. Process for the preparation of compounds of formula (I) according to claim 1, characterised in that a compound of formula (II): 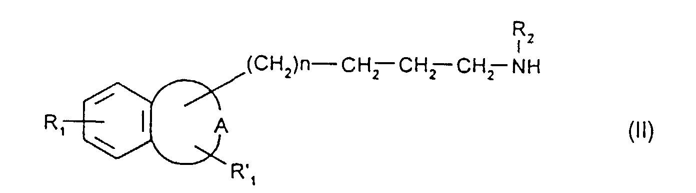 wherein R₁, R'₁, R₂, n and A are as defined in claim 1, is reacted either
with a compound of formula (IIIa) or (IIIb): 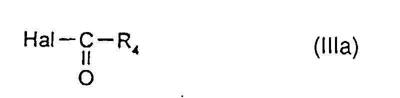 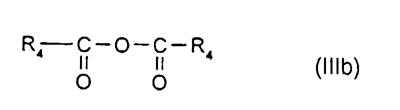 the anhydride of formula (Illb) being mixed or symmetric,
wherein R₄ is as defined in claim 1 and Hal represents a halogen atom, in order to obtain the compounds of formula (I/a): 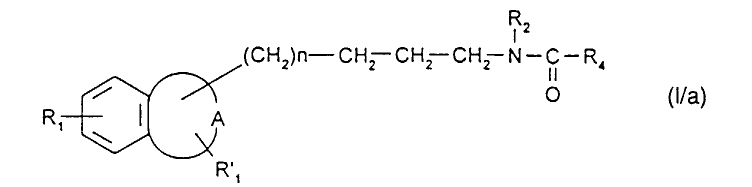 wherein R₁, R'₁, R₂, R₄, A and n are as defined hereinbefore,
which compounds of formula (I/a) are subjected to the action of a thionisation agent, for example Lawesson's reagent, to obtain the compounds of formula (I/a'): 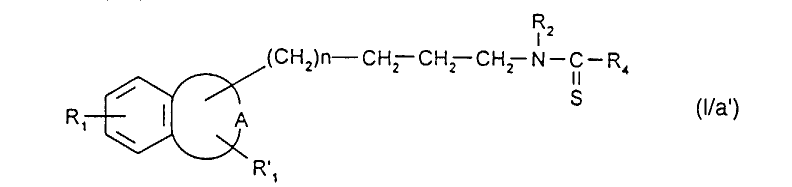 wherein R₁, R'₁, R₂, R₄, A and n are as defined hereinbefore,
or
- with a compound of formula (IV):
X'=C=N-R₅ (IV)
wherein X^{'} and R₅ are as defined in claim 1,
in order to obtain the compounds of formula (I/b): 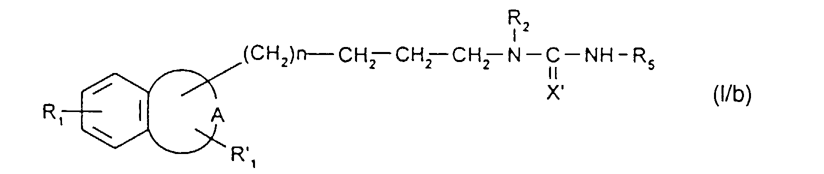 wherein R₁, R'₁, R₂, R₅, A, n and X' are as defined hereinbefore, the compounds of formulae (I/a), (I/a') and (I/b) constituting the totality of the compounds of formula (I) according to claim 1, which may, if desired, be:
- purified according to one or more methods of purification selected from crystallisation, chromatography over silica gel, extraction, filtration, and passage over charcoal or resin,
- separated, where appropriate, in pure form or in the form of a mixture, into their possible enantiomers or diastereoisomers, or
- converted into salts with a pharmaceutically acceptable base.

9. Pharmaceutical compositions comprising the compounds of formula (I) according to claim 1 or an addition salt thereof with a pharmaceutically acceptable base in combination with one or more pharmaceutically acceptable excipients.

10. Compositions according to claim 9 for use in the treatment of disorders of the melatoninergic system.
